# EUROPÄISCHE PATENTSCHRIFT

(11) **EP 1 874 821 B1**
(45) Veröffentlichungstag und Bekanntmachung des Hinweises auf die Patenterteilung: **17.04.2013**
(21) Anmeldenummer: 05738161.8
(22) Anmeldetag: 26.04.2005
(51) Int. Cl.: C07K 16/30, C07K 16/28, A61K 31/573, A61K 39/00, A61P 35/00

(54) **KOMBINATION VON ANTIKÖRPERN MIT GLUKOKORTIKOIDEN ZUR BEHANDLUNG VON KREBS**
COMBINATION OF ANTIBODIES AND GLUCOCORTICOIDS FOR TREATING CANCER
COMBINAISON D'ANTICORPS ET DE GLUCOCORTICOIDES DESTINEE AU TRAITEMENT DU CANCER

(43) Veröffentlichungstag der Anmeldung: 09.01.2008
(73) Patentinhaber: TRION PHARMA GMBH, 80807 München (DE)
(72) Erfinder: LINDHOFER, Horst, 80639 München (DE); HEISS, Markus, M., 82343 Pöcking (DE)
(74) Vertreter: Graf von Stosch, Andreas
(86) Internationale Anmeldenummer: PCT/EP2005/004468
(87) Internationale Veröffentlichungsnummer: WO 2006/114115

(56) Entgegenhaltungen:
- WO-A-02/064634
- WO-A-2004/071404
- WO-A-2004/105737
- WO-A-2005/027839
- WO-A1-01/34194
- WO-A1-02/04021
- WO-A2-03/030835
- WO-A2-03/043583
- US-A- 6 030 615
- US-A1- 2002 012 665
- FUKUDA TAKAHIRO ET AL: "Outcomes of graft failure/rejection following allogeneic hematopoietic cell transplantation: 10-Year experience at FHCRC." BLOOD, Bd. 102, Nr. 11, 16. November 2003 (2003-11-16), Seite 240a, XP008057118 & 45TH ANNUAL MEETING OF THE AMERICAN SOCIETY OF HEMATOLOGY; SAN DIEGO, CA, USA; DECEMBER 06-09, 2003 ISSN: 0006-4971
- METZLER BERNHARD ET AL: "Inhibition of arteriosclerosis by T-cell depletion in normocholesterolemic rabbits immunized with heat shock protein 65" ARTERIOSCLEROSIS THROMBOSIS AND VASCULAR BIOLOGY, Bd. 19, Nr. 8, August 1999 (1999-08), Seiten 1905-1911, XP008057116 ISSN: 1079-5642

## Beschreibung

Die vorliegende Erfindung betrifft die Verwendung von Glukokortikoiden zur Herstellung von Arzneimitteln, welche die aufgrund einer unspezifischen Zytokinfreisetzung auftretenden unerwünschten Nebenwirkungen bei der Behandlung von Erkrankungen mit immunstimulierenden Antikörpern vermindern oder beseitigen, sowie eine pharmazeutische Zusammensetzung, die mindestens einen immunstimulierenden Antikörper und mindestens ein Glukokortikoid enthält.

Immuntherapeutische Verfahren spielen bei der Behandlung bisher nur schwierig oder unzureichend kontrollierbarer Erkrankungen, insbesondere Krebs und Infektionen, eine zunehmende Rolle. Bevorzugt werden dabei Antikörper als immunstimulierende Mittel verwendet; vgl. Glennie und Johnson (2000) Immunology Today 21(8): 403-410. Die Wirksamkeit derartiger immunstimulierender Antikörper basiert insbesondere auf der Möglichkeit der spezifischen Bindung definierter Antigene. Ein großes Problem bei der Bekämpfung von Erkrankungen, wie Krebs und Infektionen, mittels immunstimulierender Antikörper waren jedoch bisher die mit der Applikation derartiger Antikörper hervorgerufenen schweren Nebenwirkungen, wobei insbesondere starkes Unwohlsein, Erbrechen, allergische Reaktionen, Hypotonie, Tachykardie, hohes Fieber und sogar tödliches Kreislauf- bzw. Organversagen meist aufgrund eines SIRS (engl. "systemic inflammatory response syndrome") beobachtet wurden.

Diese starken Nebenwirkungen von immunstimulierenden Antikörpern korrelieren in den meisten Fällen mit einer von den Antikörpern hervorgerufenen unspezifischen Zytokinfreisetzung, die für einen Großteil der genannten klinischen Nebenwirkungen verantwortlich ist. Das Ausmaß der Zytokinfreisetzung, das von der applizierten Menge und von der Applikationsgeschwindigkeit des immunstimulierenden Antikörpers abhängig ist, limitiert die verträgliche Dosis des jeweiligen immunstimulierenden Antikörpers in der klinischen Anwendung.

Glukokortikoide sind seit langem als hochwirksame antiinflammatorische und immunsuppremierende Wirkstoffe bekannt. Ein Mechanismus im Zusammenhang mit der immunsuppremierenden Wirkung von Glukokortikoiden ist deren vermindernde Wirkung auf die Transkription von Zytokinen; vgl. bspw. Blotta et al. (1997) J. Immunol. 158: 5589 bis 5595; Ballow und Nelson (1997) JAMA 278 (22), Kapitel 24: 2008 bis 2017. In den letzten Jahren wurden weitere Fortschritte hinsichtlich der Erforschung der immunsuppremierenden Wirkung von Glukokortikoiden, insbesondere im Zusammenhang mit der Entwicklung von T-Zellen und deren Funktion, gemacht; zusammenfassend dargestellt bspw. in Ashwell und Vacchio (2000) Annu. Rev. Immunol. 18: 309 bis 345. Bei der Entwicklung wirksamer immunsupprimierender Therapien, insbesondere bei der Verhinderung (akuter) Transplantatabstoßungen, werden Glukokortikoide in Kombination mit weiteren immunsupprimierenden Agenzien bevorzugt verwendet. So berichten bspw. Herbelin et aL (Transplantation (2000) 68 (5): 616 bis 622) von einer Verbesserung der akuten Immunsuppressionstherapie mit dem Anti-CD3-Antikörper OKT3 bei vorheriger Gabe von Glukokortikoiden aufgrund der durch diese hervorgerufene Verminderung der Produktion von TNF-α, IL-2 und IFN-γ. Im Stand der Technik ist allerdings eine Gabe von Glukokortikoiden im Zusammenhang mit der Stimulation des Immunsystems eines Patienten durch Antikörpertherapien, beispielsweise mit trifunktionellen Antikörpern (trAK), völlig unbekannt. Insbesondere sind Glukokortikoide bisher dann eingesetzt worden, wenn eine Immunsuppression das therapeutische Ziel war.

Der vorliegenden Erfindung liegt daher die Aufgabe zugrunde, ein neues System zur möglichst weitgehenden Verminderung der Nebenwirkungen immunstimulatorisch wirkender Antikörper bereitzustellen.

Diese Aufgabe wird durch die in den Ansprüche gekennzeichneten Ausführungsformen der vorliegenden Erfindung gelöst.

Insbesondere wird erfindungsgemäß die Verwendung eines oder mehrerer Glukokortikoide zur Herstellung eines Arzneimittels zur Verminderung der unspezifischen Zytokinfreisetzung bei der Behandlung von Erkrankungen mit einem oder mehreren immunstimulierenden Antikörpern gemäss der Ansprüche bereitgestellt.

Diese neuartige Indikation von Glukokortikoiden im Zusammenhang mit der Antikörpertherapie zur Immunstimulation bei Erkrankungen, insbesondere Krebs, beruht auf der überraschenden Feststellung, dass die Kombination von immunstimulierenden Antikörpern mit definierter Spezifität zusammen mit Glukokortikoiden zu einer Reduktion der unspezifischen Zytokinfreisetzung durch immunologische Zellen führt, ohne dass die gegen das bzw. die definierte(n) Antigen(e) gerichtete Wirkung der immunstimulierenden Antiköper beeinträchtigt wird. Insbesondere im Zusammenhang mit dem Einsatz von Antikörpern zur Immunstimulation (bspw. gegen Tumorzellen) führt die Kombination der Antikörper mit Glukokortikoiden zu einer Modulation der resultierenden Immunaktivität: Die gegen das oder die definierte(n) Antigen(e) gerichtete Immunaktivität des Antikörpers/der Antikörper bleibt weitgehend unverändert, während unspezifische Effekte, wie die vorstehend beschriebene unspezifische Zytokinfreisetzung, die bei der Applikation immunstimulierender Antikörper unabhängig von der Bindung an das oder die Zielantigen(e) auftreten, signifikant vermindert werden.

Aufgrund der Kombination von Glukokortikoiden (d.h. eines oder mehrerer) mit einem oder mehreren immunstimulierenden Antikörpern ergeben sich gegenüber den im Stand der Technik bekannten immunstimulatorischen Therapien mehrere Vorteile, welche die Möglichkeiten, insbesondere der klinischen Anwendbarkeit, von Antikörpern zur Immuntherapie erheblich erweitern:
1. Durch die Verminderung der Zytokinfreisetzung treten bei gleicher Dosierung und weitgehend unveränderter spezifischer Wirkung (d.h. gegen das oder die ZielAntigen(e) gerichtet) signifikant geringere unerwünschte Nebenwirkungen (wie vorstehend aufgeführt) auf.
2. Durch die Verminderung der unerwünschten Nebenwirkungen kann die Dosierung des oder der zu applizierenden Antikörper erheblich gesteigert werden. Dadurch ist in Kombination mit Glukokortikoiden eine erhebliche Wirkungssteigerung über die Erhöhung der Antikörper-Dosis möglich.
3. Überraschenderweise vermindern die erfindungsgemäß einzusetzenden Glukokortikoide nur die unspezifische, von der Bindung des Zielantigens unabhängige (systemische) Zytokinfreisetzung. Die Freisetzung von Zytokinen tritt daher nur am Ort der Antigen-Bindung (bspw. am Ort der Bindung und gewollten Zerstörung von Tumorzellen) auf. Daher wird die Wirkung der immunstimulierenden Antikörper aufgrund dieses Effekts zusätzlich zu der vorstehend dargelegten nunmehr möglichen, höheren Dosierung an den Ort der Antigen-Bindung fokussiert, ohne von systemisch freigesetzten Zytokinen beeinflusst zu werden.

Der Begriff "Antikörper" umfasst i.S. der vorliegenden Erfindung monoklonale Antikörper, chimäre Antikörper, humanisierte Antikörper, die alle in gebundener oder löslicher Form vorliegen können, sowie auch Fragmente der vorgenannten Antikörper. Neben den Fragmenten von erfindungsgemäßen Antikörpern in Alleinstellung können erfindungsgemäße Antikörper auch in rekombinanter Form als Fusionsproteine mit anderen (Protein)-Bestandteilen auftreten. Fragmente als solche oder Fragmente von erfindungsgemäßen Antikörpern als Bestandteile von Fusionsproteinen werden typischerweise durch die Methoden enzymatischer Spaltung, der Protein-Synthese oder die dem Fachmann geläufigen Rekombinationsmethoden hergestellt. Gemäss der vorliegenden Erfindung ist es ein trifunktioneller Antikörper (trAK).

Bei den erfindungsgemäßen chimären Antikörpern handelt es sich um Moleküle, gemäss der Ansprüche die verschiedene Bestandteile enthalten, wobei diese sich aus verschiedenen Tierarten ableiten (z. B. Antikörper, die eine variable Region, die aus einem Mäuse-monoklonalen Antikörper abgeleitet ist, und eine konstante Region eines humanen Immunglobulins aufweisen). Chimäre Antikörper gemäss der Ansprüche werden vorzugsweise eingesetzt, um einerseits die Immunogenizität bei der Anwendung zu reduzieren und andererseits die Ausbeuten bei der Produktion zu erhöhen, z.B. ergeben murine monoklonale Antikörper höhere Ausbeuten aus Hybridom-Zellinien, führen aber auch zu einer höheren Immunogenizität beim Menschen, so dass human/murine chimäre Antikörper vorzugsweise eingesetzt werden. Noch mehr bevorzugt ist ein monoklonaler Antikörper, der die hypervariablen, Komplementaritäts-bestimmenden Regionen (CDR, engl. "complementarity defining region") eines murinen monoklonalen Antikörpers mit den übrigen Bereichen eines humanen Antikörpers in sich vereinigt. Ein derartiger Antikörper wird humanisierter Antikörper genannt. Chimäre Antikörper und Verfahren zu ihrer Herstellung sind aus dem Stand der Technik bekannt (Cabilly et al., Proc. Natl. Sci. USA 81: 3273-3277 (1984); Morrison et aL Proc. Natl. Acad. Sci USA 81:6851-6855 (1984); Boulianne et aL Nature 312 643-646 (1984); Cabilly et aL, EP-A-125023; Neuberger et aL, Nature 314: 268-270 (1985); Taniguchi et aL, EP-A-171496; Morrion et al., EP-A-173494; Neuberger et aL, WO 86/01533; Kudo et aL, EP-A-184187; Sahagan et al., J. Immunol. 137: 1066-1074 (1986); Robinson et al., WO 87/02671; Liu et aL, Proc. NatL Acad. Sci USA 84: 3439-3443 (1987); Sun et aL, Proc. Natl. Acad. Sci USA 84: 214218 (1987); Better et aL, Science 240: 1041-1043 (1988) und Harlow und Lane, Antibodies: A Laboratory Manual, *supra*.

Der Begriff "immunstimulierender Antikörper" oder "immuntherapeutischer Antikörper" im Sinne der vorliegenden Erfindung bedeutet, dass der jeweilige Antikörper aufgrund seiner Antigen-Spezifität eine im Rahmen der Behandlung der jeweiligen Erkrankung erwünschte Stimulation des Immunsystems des Patienten hervorruft oder unterstützt.

Insbesondere sind immunstimulierende Antikörper im Sinne der vorliegenden Erfindung solche, die eine T-Zell-Aktivierung hervorrufen. Besonders ist dabei eine Aktivierung von zytotoxischen T-Zellen (CTL, engL "cytotoxic T lymphocytes", sog. T-Killerzellen) vorteilhaft. Ebenfalls umfasst sind jedoch auch immunstimulierende Antikörper mit Antikörpervermittelten Effekten, die beispielsweise über eine Aktivierung von T-Helferzellen, akzessorischen Zellen (Makrophagen), dendritischen Zellen, B-Zellen oder natürlichen Killerzellen (NK-Zellen) erfolgen.

Immunstimulatorische Antikörper sind bispezifisch, und trifunktionell. Insbesondere bei bispezifischen Antikörpern sind rekombinante Antikörpermoleküle zu nennen, die durch rekombinante Techniken hergestellt werden, bspw. scFv-Moleküle (sog. "single chain antibodies"), Diabodies usw. Der grundsätzliche Aufbau bispezifischer Antikörper und Immunkonjugate ist bspw. in van Spriel et aL (2000) Immunol. Today 21: 391-397, dargestellt. Bispezifische Antikörper können selbstverständlich auch durch bekannte Hybridom-Techniken hergestellt werden. Verfahren zur Herstellung multivalenter und bispezifischer Antikörperfragmente sind einem Fachmann bekannt und z.B. in Tomlinson und Holliger (2000) Meth. EnzymoL 326: 461 ff., beschrieben. Ein besonders bevorzugtes Beispiel eines bispezifischen Antikörpers ist ein trifunktioneller bispezifischer Antikörper, an dessen Fc-Teil, d.h. der Teil des Antikörpers, der nicht direkt an der Antigenbindung beteiligt ist, akzessorische Immunzellen binden können.

Bevorzugte bispezifische immunstimulatorische Antikörper sind erfindungsgemäß solche, die mindestens eine Spezifität gegen ein Antigen auf einer abzutötenden Zelle, bspw. einer Tumorzelle und gegen einen CD-Marker aufweisen. Der CD-Marker eines derartigen bispezifischen, immunstimulierenden Antikörpers wird bevorzugt auf T-Lymphozyten exprimiert und ist daher aus der Gruppe, bestehend aus CD2, CD3, CD4, CD5, CD6, CD8, CD28 und CD44 ausgewählt. Die T-Zell-Spezifität des bispezifischen Antikörpers rekrutiert somit zum einen in spezifischer Weise T-Zellen. Die zweite Spezifität des bevorzugten, immunstimulatorisch wirksamen Antikörpers ist gegen ein Antigen gerichtet, das möglichst spezifisch auf der durch das Immunsystem zu eliminierenden Zelle exprimiert wird, gerichtet. Bei Krebszellen ist dies vorzugsweise ein sog. Tumorantigen, d.h. ein auf der Oberfläche der Zellen eines Tumors exprimiertes Peptid bzw. Polypeptid. Bevorzugte Tumorantigene immunstimulatorisch gegen Krebszellen wirksamer Antikörper sind bspw. EpCAM, HER2/neu, HER3/neu, G250, CEA, MAGE, VEGF, GD3, EGFR, αVβ3-Integrin, HLA, HLA-DR, ASC, CD1, CD2, CD4, CD6, CD7, CD8, CD11, CD13, CD14, CD19, CD20, CD21, CD22, CD23, CD24, CD33, CD40, CD41, CD52, c-erb-2, CALLA (CD10), MHCII, CD44v3, CD44v6, CD117, p97, GangliosidGD2, GD3, C215, Antigen des Ak 9.2.27, Antigen des Ak NE150 und Antigen des Ak CA125 (vgL auch Jager (2001) J. Clin. Pathol. 54(9): 669-674; Jager (2002) Curr. Opin. ImmunoL 14(2): 178-182).

In diesem Zusammenhang besonders bevorzugt ist, wie bereits erwähnt, eine Variante eines bispezifischen Antikörpermoleküls, die am Fc-Teil ein oder mehrere Bindungsstellen für akzessorische Immunzellen aufweist. Dieser Antikörpertyp rekrutiert also nicht nur die zu eliminierende Zelle, bspw. eine Tumorzelle, und T-Zellen, sondern gleichzeitig auch akzessorische Immunzellen wie Monozyten oder Makrophagen und formiert so einen "Tri-Zell-Komplex". Durch die Rekrutierung des dritten zellulären Bindungspartners wird die Phagozytose von der zu eliminierenden Zelle ausgelöst. Dies wiederum ist Voraussetzung für die Entstehung einer polyklonalen Immunantwort, wodurch der Patient nicht nur gegen das isolierte, auf der zu eliminierenden Zelle exprimierte Antigen immunisiert wird, sondern gegen eine Vielzahl der zu eliminierenden Zellen, insbesondere Tumorzellen, im Körper des Patienten mit allen eventuellen Mutationen.

Besonders bevorzugte Ausführungsformen trifunktional bispezifischer Antikörper, die erfindungsgemäß immunstimulatorisch wirksam sind, sind bspw. mit der Spezifität anti-EpCAM x anti-CD3 oder anti-HER2/neu x anti-CD3 ausgestattet.

Das erfindungsgemäß zu verwendende Glukokortikoid ist nicht besonders eingeschränkt und kann sowohl natürlich vorkommend, bspw. Hydrocortison (Cortisol) oder Cortison, oder synthetischer Natur sein. Auch bezüglich einer Auswahl hinsichtlich der biologischen Halbwertszeit (kurz wirksam, intermediär wirksam und lang wirksam) besteht keinerlei Einschränkung. Erfindungsgemäß zu verwendende synthetische Glukokortikoide sind bspw. Prednison, Prednisolon, Methylprednisolon, Triamcinolon Betamethason, Dexamethason, Cortisonacetat, Prednyliden, Deflazacort, Cloprednol, Fluocortolon und Budenosid, wobei diese Aufzählung keineswegs abschließend ist.

Ein besonders bevorzugtes Glukokortikoid gemäß der vorliegenden Erfindung ist das Glukokortikoid Dexamethason. Es handelt sich hierbei um ein synthetisches Glukokortikoid, dessen Halbwertzeit bei 36 bis 54 Stunden liegt; dies entspricht einem lang wirksamen Glukokortikoid. Es ist daher besonders für eine Behandlung mit erforderlicher kontinuierlicher Glukokortikoidwirkung geeignet. Dexamethason wird nach dessen Verabreichung zu 70 bis 80 % an Albumin gebunden, so dass ein freier und wirksamer Anteil des Glukokortikoids von 20 bis 30 % vorliegt. Dexamethason besitzt eine ungefähr 30-fach stärkere Wirkung als das natürliche Nebennierenrindenhormon Kortison, eine geringe Mineralokortikoidwirkung und eine geringe wasser- und salzretinierende Wirkung. Es ist unter vielen anderen Anwendungen insbesondere auch für die Applikation bei Patienten mit Herzinsuffizienz oder Hypertonie geeignet. Therapeutisch von Bedeutung ist weiterhin die starke antiphlogistische und immunsuppresive (anti-allergische) Wirkung von Dexamethason. Ebenfalls vorteilhaft ist, dass nach Injektion i.v. innerhalb weniger Minuten maximale Plasmakonzentrationen erreicht werden.

Als Beispiele für mit dem erfindungsgemäßen immuntherapeutischen Antikörper (oder auch mehrerer Antikörper) in Verbingung mit dem Glukokortikoid (oder mehreren Glukokortikoiden) behandelbare Krebserkrankungen können Magenkarzinom, Adenokarzinom, malignes Melanom, Kolonkarzinom, Pankreaskarzinom, Ovarialkarzinom, Uteruskarzinom, Hepatozelluläres Karzinom, Bronchialkarzinom (alle histologische Typen), Lymphome, Sarkome, kleinzelliges Lungenkarzinom, Blastome, gastrointestinaler Stromatumor (GIST) usw., genannt werden.

Hinsichtlich der Art und Weise der Verabreichung sowohl des immunstimulatorischen Antikörpers als auch des Glukokortikoids bestehen erfindungsgemäß keinerlei Einschränkungen. Daher kann sowohl das Glukokortikoid als auch der Antikörper intraperitoneal, systemisch (intravenös oder intraarteriell), intramuskulär, intradermal, subkutan, intratumoral oder aber auch selektiv in bzw. über ein definiertes Organ verabreicht werden. Selbstverständlich kann das Glukokortikoid insbesondere auch oral oder, in Form einer Salbe, eines Gels oder einer anderen geeigneten Darreichungsform auch auf die Haut aufgetragen werden. Als Beispiel einer selektiven Applikation in ein oder über ein Organ kann die Verabreichung über das Knochenmark (als immunologisches Organ) oder über einen superselektiven Katheter in eine das jeweilige Organ versorgendes Gefäß (Arterie) genannt werden. Ein spezifisches Beispiel einer derartigen Applikation mittels Katheter kann diejenige in die A. hepatica zur selektiven Applikation in die Leber bzw. zur systemischen Verabreichung nach Durchlaufen des Organs angegeben werden. Weitere Beispiele der organspezifischen Applikation sind diejenige in die Leber über die Pfortader, in die Niere über die Nierenarterie, intrathekale Applikation bei cerebralen Tumoren, in den Colonbereich über Mesentarialgefäße, in den Pankreas über den Truncus coeliacus und die A. mesenteria superior und in Tumoren an Gliedmaßen über die entsprechenden Arterien. Des weiteren kann auch eine direkte Applikation in einen Tumor erfolgen. Selbstverständlich kann bei der erfindungsgemäßen Verwendung immunstimmulierender Antikörper und Glukokortikoid die Applikation dieser wirksamen Komponenten auf gleichen oder auf verschiedenen Wegen erfolgen, bspw. der bzw. die Antikörper selektiv über die Leber, das oder die Glukokortikoid(e) systemisch, z.B. intravenös.

Das Glukokortikoid kann bspw. gleichzeitig, getrennt oder zeitlich abgestuft zu dem als immunstimulatorischen Mittel wirkenden Antikörpers verabreicht werden. Das Glukokortikoid kann demnach zeitlich vor oder nach oder gleichzeitig zu dem Antikörper verabreicht werden. Gemäß einer besonders bevorzugten Ausführungsform werden das Glukokortikoid und der immunstimulatorische Antikörper etwa gleichzeitig verabreicht.

Ein weiterer Gegenstand der Erfindung ist ein Erzeugnis, enthaltend mindestens einen wie vorstehend definierten immunstimulatorischen bzw. immuntherapeutischen Antikörper und mindestens ein Glukokortikoid gemäß obiger Definition als Kombinationspräparat zur gleichzeitigen, getrennten oder zeitlich abgestuften Anwendung bei der Behandlung und/oder Prophylaxe von Krebserkrankungen, Tumorerkrankungen. Krebserkrankungen umfassen beispielsweise Magenkarzinom, Adenokarzinom, malignes Melanom, Kolonkarzinom, Pankreaskarzinom, Ovarialkarzinom, Uteruskarzinom, Hepatozelluläres Karzinom, allen histologischen Typen des Bronchialkarzinoms, Lymphomen, Sarkomen und/oder Blastomen.

Die Bestandteile des erfindungsgemäßen Erzeugnises: mindestens einen wie vorstehend definierten immunstimulatorischen bzw. immuntherapeutischen Antikörper (1. Bestandteil) und mindestens ein Glukokortikoid gemäß obiger Definition (2. Bestandteil), stehen durch ihre zielgerichtete Verwendung in funktioneller Einheit. Die Bestandteile des Erzeugnisses können die oben beschriebene, erfindungsgemäße, vorteilhafte Wirkung nicht unabhängig voneinander entfalten, so dass trotz der räumlichen Trennung der Bestandteile 1 und 2 (zur gleichzeitigen, getrennten oder zeitlich abgestuften Verabreichung) ihre Anwendung als neues, nicht im Stand der Technik beschriebenes Kombinationserzeugnis vorliegt.

Ein erfindungsgemäßes Erzeugnis kann alle Bestandteile, Substanzen und Ausführungsformen umfassen, wie sie in einem Verfahren bzw. Therapieverfahren bzw. Verfahren zur Behandlung und/oder Prophylaxe von Erkrankungen bzw. Kombinationstherapieverfahren gemäß der vorliegenden Erfindung eingesetzt werden.

Ein weiterer Gegenstand der Erfindung betrifft eine pharmazeutische Zusammensetzung, enthaltend mindestens einen wie vorstehend definierten immunstimulatorischen bzw. immuntherapeutischen Antikörper und mindestens ein Glukokortikoid gemäß obiger Definition. Die pharmazeutische Zusammensetzung der vorliegenden Erfindung eignet sich insbesondere zur Behandlung der vorstehend aufgeführten Erkrankungen. Gegebenenfalls liegen die pharmakologisch wirksamen Bestandteile der erfindungsgemäßen pharmazeutischen Zusammensetzung in Verbindung mit einem oder mehreren Trägern und/oder Hilfsstoffen vor, wie nachstehend genauer dargelegt wird.

In der erfindungsgemäßen pharmazeutischen Zusammensetzung bzw. bei der erfindungsgemäßen Verwendung der Glukokortikoide werden diese und der Antikörper vorteilhafterweise in geeigneten Formulierungen bereitgestellt. Derartige Formulierungen sind einem Fachmann bekannt und enthalten neben den therapeutisch bzw. immunstimulatotisch wirkenden Substanzen einen oder mehrere pharmazeutisch verträgliche Träger und/oder pharmazeutisch verträgliche Vehikel. Entsprechende Wege zur geeigneten Formulierung und Herstellung derartiger Formulierungen sind bspw. bei "Remington's Pharmaceutical Sciences" (Mack Pub. Co., Easton, PA, 1980) offenbart, das vollinhaltlich Bestandteil der Offenbarung der vorliegenden Erfindung ist. Für die paranterale Verabreichung kommen als Trägerstoffe bspw. steriles Wasser, sterile Kochsalzlösung, Polyalkylenglykole, hydrierte Naphthalene und insbesondere biokompatible Lactidpolymere, Lactid/Glykolidcopolymere oder Polyoxyethylen-/Polyoxypropylencopolymere in Betracht. Erfindungsgemäße pharmazeutische Zusammensetzungen können Füllsubstanzen oder Substanzen, wie Laktose, Manitol, Substanzen zur kovalenten Anknüpfung von Polymeren, wie z.B. Polyethylenglykol an erfindungsgemäße immunstimulatorische Antikörper, Komplexierung mit Metallionen oder Einschluß von Materialien in oder auf besondere Präparationen von Polymerverbindungen, wie z.B. Polylaktat, Polyglykolsäure, Hydrogel oder auf Liposomen, Mikroemulsionen, Mizellen, unilamerare oder multilamelare Vesikel, Erythrozyten-Fragmente oder Spheroblasten, enthalten. Die jeweiligen Ausführungsformen der pharmazeutischen Zusammensetzungen werden abhängig vom physikalischen Verhalten, bspw. in Hinblick auf die Löslichkeit, die Stabilität, Bioverfügbarkeit oder Abbaubarkeit gewählt. Kontrollierte oder konstante Freisetzung der erfindungsgemäßen Wirkstoffkomponenten schließt die Formulierung auf Basis lipophiler Depots ein (z.B. Fettsäuren, Wachse oder Öle). Im Rahmen der vorliegenden Erfindung werden auch Beschichtungen erfindungsgemäßer pharmazeutischer Zusammensetzungen bzw. Arzneimittel, enthaltend die therapeutisch wirksamen Substanzen, nämlich Beschichtungen mit Polymeren offenbart (z.B. Polyoxamere oder Polyoxamine). Weiterhin können erfindungsgemäße therapeutisch wirksame Substanzen oder Zusammensetzungen protektive Beschichtungen, z.B. Proteaseinhibitoren oder Permeabilitätsverstärker, aufweisen. Bevorzugte Träger sind typischerweise wässrige Trägermaterialien, wobei Wasser zur Injektion (WFI) oder Wasser, gepuffert mit Phosphat, Zitrat, HEPES oder Acetat usw. verwendet wird und der pH typischerweise auf 5,0 bis 8,0 (vorzugsweise 6,5 bis 7,5) eingestellt wird. Der Träger bzw. das Vehikel wird zusätzlich vorzugsweise Salzbestandteile enthalten, z.B. Natriumchlorid, Kaliumchlorid oder andere Komponenten, welche die Lösung bspw. isotonisch machen. Weiterhin kann der Träger bzw. das Vehikel neben den vorstehend genannten Bestandteilen zusätzliche Komponenten, wie humanes Serumalbumin (HSA), Polysorbat 80, Zucker oder Aminosäuren usw., enthalten.

Die Art und Weise der Verabreichung und die Dosierung des erfindungsgemäßen Arzneimittels bzw. des erfindungsgemäßen Erzeugnisses bzw. der pharmazeutischen Zusammensetzungen hängen von der Art der zu bekämpfenden Erkrankung, ggf. deren Stadium, dem anzusteuernden Antigen wie auch dem Körpergewicht, dem Alter und dem Geschlecht des Patienten ab.

Die Konzentration der wirksamen Komponenten in den erfindungsgemäßen Formulierungen kann innerhalb eines weiten Bereichs variiert werden. Erfindungsgemäße Dosen des Antikörpers schwanken im Bereich von etwa 1 µg bis etwa 1 mg, während Glukokortikoide im allgemeinen in Dosen von etwa 1 mg bis etwa 1000 mg, insbesondere etwa 1 mg bis etwa 100 mg, verabreicht werden.

Erfindungsgemäß wird des weiteren ein Verfahren zur Behandlung der vorstehend genannten Erkrankungen bereitgestellt, welches das Verabreichen eines immunstimulierenden Antikörpers gemäß vorstehender Definition und mindestens einen Glukokortikoids an einen Patienten, insbesondere einen Menschen, umfasst. Dabei ist die vorherige oder gleichzeitige Applikation des Glukokortikoids vor bzw. zusammen mit dem immunstimulierenden Antikörper bevorzugt. Selbstverständlich können erfindungsgemäß mehrere immunstimulierende Antikörper und mehrere Glukokortikoide in jeglicher Kombination verwendet werden.

Ein weiterer Gegenstand der Erfindung betrifft ein Kit, das mindestens einen wie vorstehend definierten immunstimulatorischen bzw. immuntherapeutischen Antikörper und mindestens ein Glukokortikoid gemäß obiger Definition enthält, wobei der mindestens eine wie vorstehend definierte immunstimulatorische bzw. immuntherapeutische Antikörper und das mindestens eine Glukokortikoid gemäß obiger Definition voneinander getrennt sind.

Eine bevorzugte Ausführungsform der Erfindung betrifft die Verwendung des Kits zur Behandlung und/oder Prophylaxe von Krebserkrankungen, Tumorerkrankungen. Krebserkrankungen umfassen beispielsweise Magenkarzinom, Adenokarzinom, malignes Melanom, Kolonkarzinom, Pankreaskarzinom, Ovarialkarzinom, Uteruskarzinom, Hepatozelluläres Karzinom, allen histologischen Typen des Bronchialkarzinoms, Lymphomen, Sarkomen und/oder Blastomen.

Die Figuren zeigen:

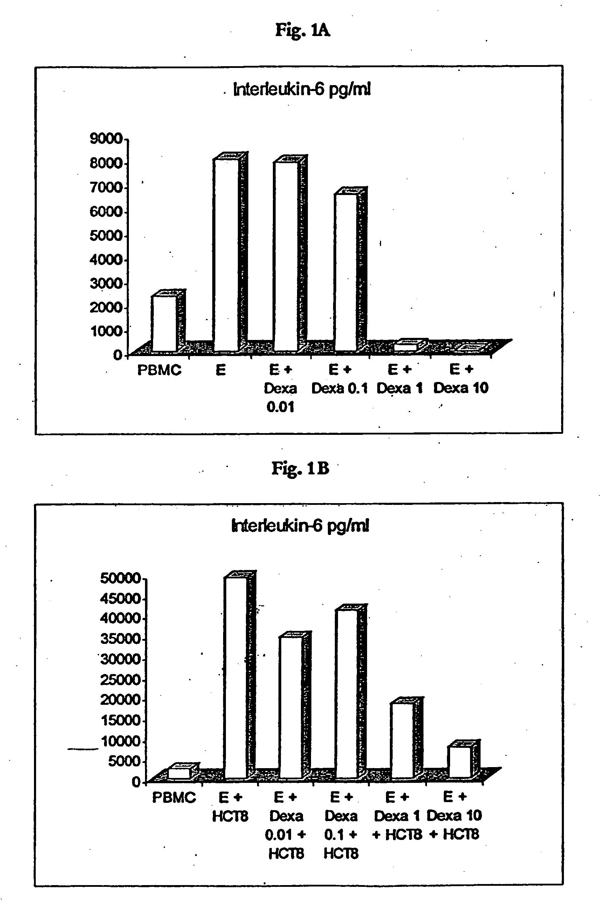

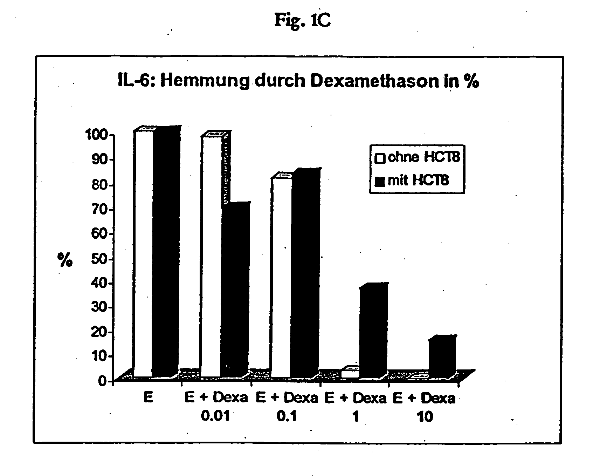

- Fig. 1: zeigt graphische Darstellungen, welche die Freisetzung von Interleukin-6 (IL-6) durch mononukleäre Zellen des peripheren Bluts (PBMC) (Konzentration 10⁶ Zellen/ml) nach einer Stimulation ohne bzw. mit EpCAM-positiven Tumorzellen (Konzentration 5 x 10⁴ Zellen/ml) dokumentieren.
- Fig. 1A: stellt die Freisetzung von IL-6 dar, wobei auf der y-Achse die IL-6-Konzentration in pg/ml aufgetragen ist. Auf der x-Achse ist die jeweilige Stimulation der PBMC aufgetragen, wobei unstimulierte PBMC als Kontrolle dienten (Balken PBMC). Dabei bedeutet E jeweils die Stimulation mit 100 µg/ml trifunktionellem bispezifischem Antikörper mit der Spezifität anti-EpCAM x anti-CD3. Mit "Dexa" wird Dexamethason mit der angegebenen Konzentration in µg/ml bezeichnet Es wurde im Experiment gemäß Fig. 1A die Freisetzung von IL-6 aus PBMC nach Stimulation ohne Kontakt zu EpCAM-positiven Tumorzellen (= Zielzellen) untersucht. Die Stimulation ohne EpCAM-positive Tumorzellen entspricht der unspezifischen Zytokinfreisetzung, die unabhängig von der Antigen-Bindung ist. Die Freisetzung erfolgt also systemisch durch die Gesamtheit der PBMC. Wie die Fig. 1A zeigt, vermindert Dexamethason die Freisetzung von IL-6 konzentrationsabhängig. Bei einer Konzentration von 1 µg/ml kommt es zu einer fast vollständigen Unterdrückung der IL-6-Freisetzung. Daher sollte sich die durch die systemische IL-6-Freisetzung hervorgerufenen Nebenwirkungen fast vollständig durch eine ausreichend hohe Dexamethason-Gabe unterdrücken lassen.
- Fig. 1B: zeigt in einer der Fig. 1A entsprechenden Auftragung die Freisetzung von IL-6 aus PBMC nach Stimulation mit Kontakt zu EpCAM-positiven Tumorzellen (HCT8; Konzentration 5 x 10⁴ Zellen/ml). Diese Vorgehensweise der Stimulation mit EpCAM-positiven Tumorzellen entspricht der Situation der Freisetzung von IL-6 am Ort der Antigen-Bindung durch die PBMC. Das in dieser experimentellen Anordnung gemessene IL-6 entspricht damit der spezifischen (d.h. iielgerichteten) Wirkung des verwendeten Antikörpers und stellt somit keine unerwünschte Nebenwirkung dar. IL-6 wird nur von direkt an der spezifischen Immunreaktion beteiligten Immunzellen freigesetzt. Die absolut höhere Freisetzung (vgl. die Einteilung der y-Achse in Fig. 1B mit derjenigen in Fig. 1A) ist daher nicht als Zeichen einer erhöhten Nebenwirkung zu werten, sondern erklärt sich aus der spezifischen immunologischen Wirkung am Ort der Antigen-Bindung. Im klinischen Einsatz des immunstimulierenden Antikörpers zusammen mit dem Glukokortikoid wird die hohe Menge an IL-6 nur von wenigen, direkt an der spezifischen Wirkung beteiligten PBMC freigesetzt. Die Nebenwirkungen ergeben sich aber aus der unspezifischen Freisetzung von Interleukinen durch die Gesamtheit der PBMC (dokumentiert in vorstehender Fig. 1A). Wie aus der Fig. 1B hervorgeht, ist bei einer Dexamethason-Konzentration von 1 µg/ml die IL-6-Freisetzung zwar vermindert, jedoch liegt sie weit oberhalb der systemischen Freisetzung, die bei einer Stimulation ohne EpCAM-positiven Tumorzellen mit dem Antikörper ohne Dexamethason beobachtet wird (vgL Fig. 1A). Daher ist bei einer Kombinationsgabe des immunstimulierenden Antikörpers mit Dexamethason die unspezifische IL-6-Freisetzung wesentlich unterdrückt, während die zielgerichtete Wirkung des immunstimulierenden Antikörpers weiterhin signifikant ist.
- Fig. 1C: stellt einen prozentualen Vergleich der IL-6-Freisetzung nach Stimulation ohne/mit HCT8-Zellen (Zielzellen) gemäß den vorstehend beschriebenen Figuren 1A und 1B in einem weiteren Diagramm dar. Auf der y-Achse ist die prozentuale IL-6-Freisetzung, bezogen auf den Wert ohne gleichzeitigen Einsatz von Dexamethason (= 100%) dargestellt. Die jeweils linken Balken stellen die Werte nach Stimulation ohne HCT8 dar, während der jeweils rechte Balken das Ergebnis nach Stimulation mit HCT8-Zellen angibt. Zwischen einer Dexamethason-Dosierung von 0,1 bis 1 µg/ml erfolgt eine Dissoziation der IL-6-Sekretion bei Stimulation der PBMC mit dem Antikörper in Abwesenheit von HCT8-Zellen im Vergleich zur Stimulation in Gegenwart von HCT8-Zellen: Bei 1 µg/ml Dexamethason bleibt die spezifische (d.h. durch Bindung der EpCAM-positiven Tumorzelle hervorgerufene) wirkungsassoziierte IL-6-Freisetzung erhalten. Im Gegensatz dazu ist die unspezifische Sekretion von IL-6, welche unabhängig von der Antigen-Bindung (im vorliegenden Fall der EpCAM-Bindung auf den HCT8-Zellen) ist, wesentlich vermindert bzw. verschwunden.

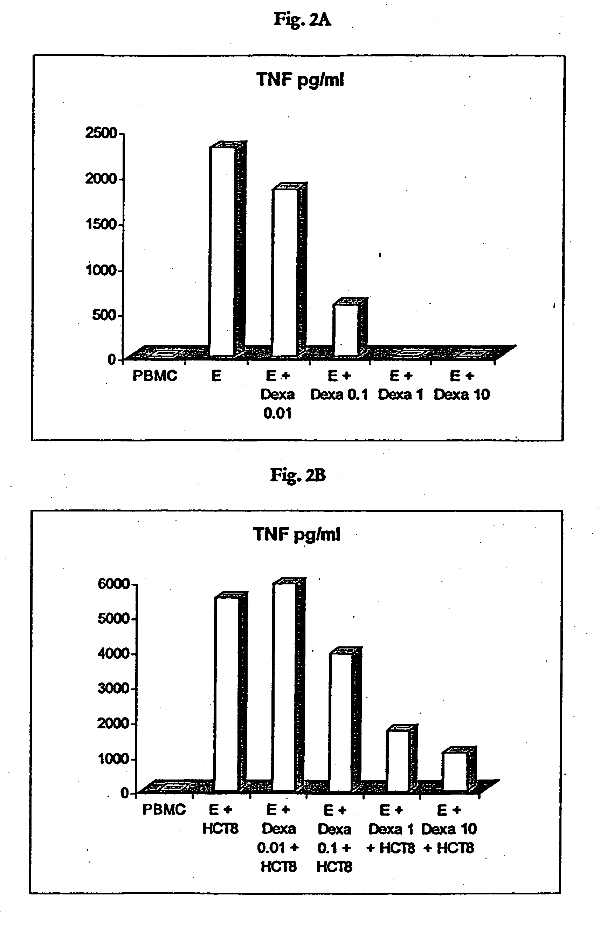

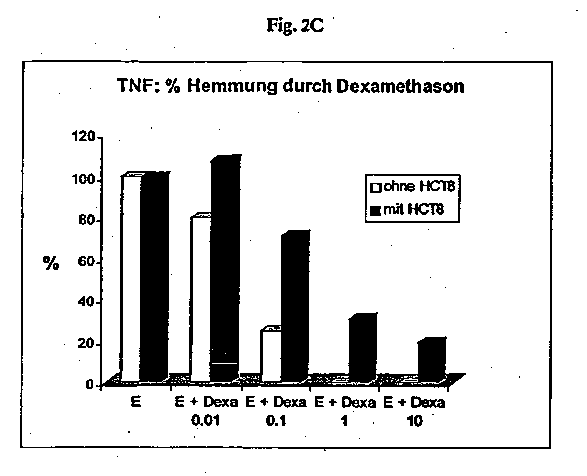

- Fig. 2: stellt in Diagrammen die Ergebnisse der Freisetzung von Tumor-Nekrose-Faktor-α (TNF-α) nach Stimulation mit trifunktionalen bispezifischem Antikörper der Spezifität anti-EpCAM x anti-CD3 ohne bzw. in Gegenwart von EpCAM-positiven Tumorzellen dar.
- Fig. 2A: zeigt in ähnlicher Weise wie die vorstehende Fig. 1A (dort bezüglich IL-6) die Freisetzung von TNF-α aus PBMC nach Stimulation ohne Kontakt zu EpCAM-positiven Tumorzellen, wobei hier auf der y-Achse die TNF-α-Konzentration in pg/ml aufgetragen ist. Diese Stimulation ohne Zielzellen entspricht wiederum, wie bereits bezüglich Fig. 1A dargelegt, der systemischen, unspezifischen Zytokin-Freisetzung, die unabhängig von der Antikörper-Antigen-Wechselwirkung auftritt Diese systemische TNF-α-Freisetzung erfolgt durch die Gesamtheit der PBMC-Population. Dexamethason vermindert die Freisetzung von TNF-α konzentrationsabhängig. Bei einer Konzentration von 0,1 µg/ml kommt es bereits zu einer starken Verminderung der TNF-α-Freisetzung, die etwa bei einem Fünftel der TNF-α-Freisetzung liegt, die bei einer Stimulation mit Antikörper ohne Glukokortikoid-Gabe auftritt. Bei einer Glukokortikoid-Konzentration ab 1 µg/ml und größer kommt es sogar zu einer vollständigen Unterdrückung der TNF-α-Freisetzung.
- Fig. 2B: stellt in einem Diagramm ähnlich wie Fig. 1B die Freisetzung von TNF-α aus PBMC nach Antikörperstimulation mit Kontakt zu EpCAM-positiven Tumorzellen dar, wobei auch hier auf der y-Achse die TNF-α-Konzentration in pg/ml aufgetragen ist. Die sonstige Auftragung entspricht derjenigen von Fig. 1B. Die in dieser experimentellen Anordnung durchgeführte Stimulation mit EpCAM-positiven Tumorzellen als Zielzellen entspricht der Situation der Freisetzung von TNF-α am Ort der Antigen-Bindung durch die PBMC. Daher ist die bei diesem Experiment gemessene TNF-α-Konzentration zum Großteil der spezifischen (zielgerichteten) Wirkung des Antikörpers zuzuschreiben und stellt somit keine unerwünschte Nebenwirkung dar. Auch bei der TNF-α-Freisetzung werden bei Stimulation mit Kontakt zu EpCAM-positiven Tumorzellen im Vergleich zur Stimulation ohne Kontakt zu Zielzellen absolut höhere Werte gemessen, die jedoch, wie schon vorstehend bezüglich der IL-6-Freisetzung bei Fig. 1B dargelegt, ihre Ursache in der spezifischen immunologischen Wirkung am Ort der Antikörper-Antigen-Wechselwirkung findet. Auch bei der vorliegend untersuchten TNF-α-Freisetzung wird bei einer Konzentration des Glukokortikoids Dexamethason von 1 µg/ml und mehr eine deutliche Verhinderung der spezifischen TNF-α-Freisetzung beobachtet, die jedoch weiterhin signifikant bleibt.
- Fig. 2C: stellt in einer der Fig. 1C entsprechenden Auftragung einen prozentualen Vergleich der TNF-α-Freisetzung nach Stimulation ohne (jeweils linker Balken) bzw. mit (jeweils rechter Balken) HCT8-Zielzellen dar. Die 100%-Werte entsprechen der TNF-α-Freisetzung ohne gleichzeitige Gabe von Dexametahson. Hinsichtlich der Auftragung vgl. Fig. 1C. Auch bezüglich TNF-α wird zwischen einer Dexamethason-Dosierung von 0,1 und 1 µg/ml eine Dissoziation der Zytokin-Sekretion ohne Zielzellen im Vergleich zur Stimulierung mit Kontakt zu Zielzellen beobachtet. Bei 1 µg/ml Dexamethason bleibt die spezifische (durch Bindung der EpCAM-positiven Tumorzelle) wirkungsassoziierte TNF-α-Freisetzung erhalten (mehr als 60% der Zytokin-Freisetzung bezogen auf die Stimulation durch Antikörper ohne gleichzeitige Glukokortikoid-Zugabe). Im Gegensatz dazu ist die unspezifische. Sekretion von TNF-α, die unabhängig von der Antigenbindung (EpCAM-Bindung auf HCT8-Zellen) auftritt, unter Zugabe von 0,1 µg/ml Dexamethason auf gut 20% des Werts vermindert, der bei Stimulation mit Antikörper ohne Dexamethason-Zugabe gemessen wird. Bei 1 mg/ml Dexamethason ist die unspezifische TNF-α-Freisetzung sogar vollständig verschwunden.

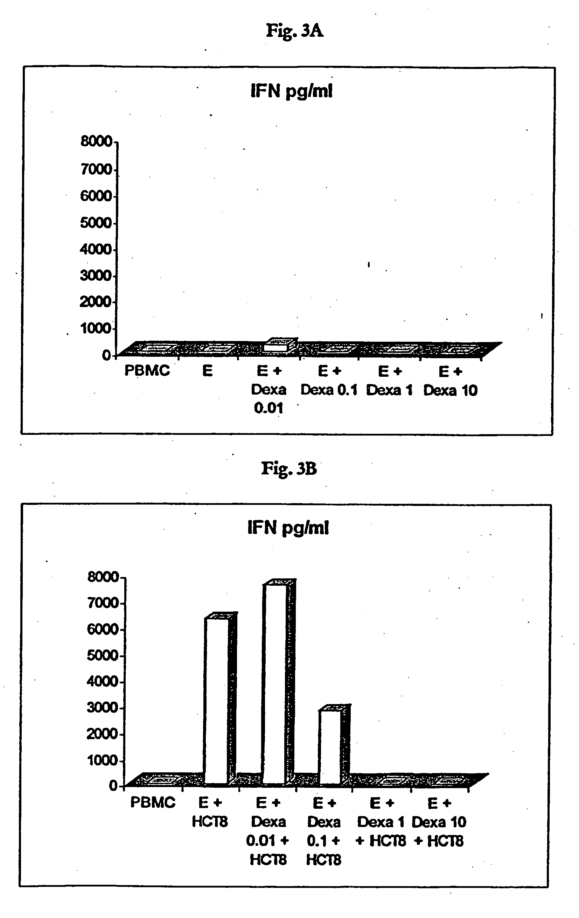

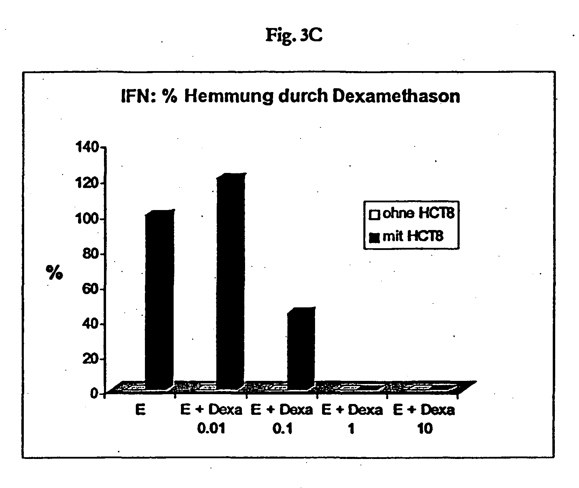

- Fig. 3: zeigt in weiteren Diagrammen, entsprechend den Darstellungen der vorstehend beschriebenen Figuren 1 und 2, Ergebnisse zur Freisetzung von Interferon-γ (IFN-γ) aus PBMC nach deren Stimulation mit bispezifischem Antikörper (anti-EpCAM x anti-CD3) ohne bzw. mit Kontakt zu Zielzellen (EpCAM-positive Tumorzellen). IFN-γ ist (wie TNF-α) ein TH1-(T-Helfer-Zelltyp 1) spezifisches Zytokin und darüber hinaus ein Marker für die T-Zell-vermittelte Antigen-spezifische Zielzell-Zerstörung. IFN-γ wird von aktivierten spezifischen T-Lymphozyten nach Stimulation gegen ein Antigen ausgeschüttet. Daher sollte IFN-γ bei einer Stimulation mit Antikörper ohne gleichzeitigen Kontakt mit den Zielzellen nicht auftreten, da hierbei keine, auf einer Zielantigen-Antikörper-Wechselwirkung beruhende Stimulation erfolgt.
- Fig. 3A: bestätigt in einer der Fig. 1A bzw. 2A entsprechenden Auftragung (y-Achse: IFN-γ-Konzentration in pg/ml), die vorstehend dargestellte Erwartung, da ohne Kontakt zu EpCAM-positiven Tumorzellen (HCT8) bei keiner Stimulation eine Freisetzung von IFN-γ erfolgt.
- Fig. 3B: dokumentiert die Freisetzung von IFN-γ aus PBMC nach Antikörper-Stimulation mit Kontakt zu EpCAM-positiven Tumorzellen (HCT8). Bei Stimulation der PBMC mit dem bispezifischen Antikörper kommt es im Kontakt zu den Zielzellen (HTC8) zu einer signifikanten Freisetzung von IFN-γ, die im Fall ohne Zugabe von Glukokortikoid bei über 6000 pg IFN-γ/ml liegt. Bei einer gleichzeitigen Gabe von 0,01 µg/ml Dexamethason ist diese IFN-γ-Freisetzung sogar noch etwas erhöht (über 7000 pg/ml). Bei einer Dexamethason-Konzentration von 0,1 µg/ml ist die IFN-γ-Freisetzung zwar auf knapp 3000 pg/ml vermindert, jedoch weiterhin signifikante.
- Fig. 3C: stellt ähnlich wie in den Figuren 1C und 2C einen prozentualen Vergleich der Zytokin-Freisetzung (hier IFN-γ) nach Stimulation durch Antikörper ohne bzw. mit Kontakt zu HCT8-Zielzellen dar.

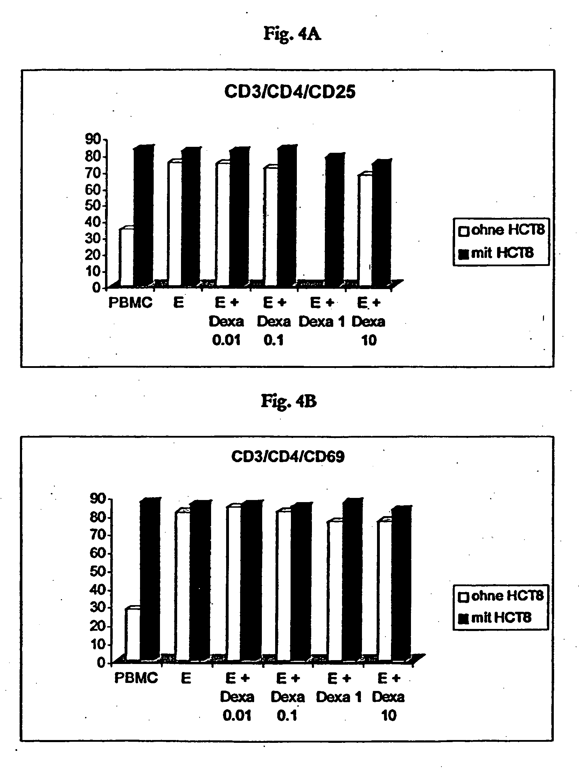

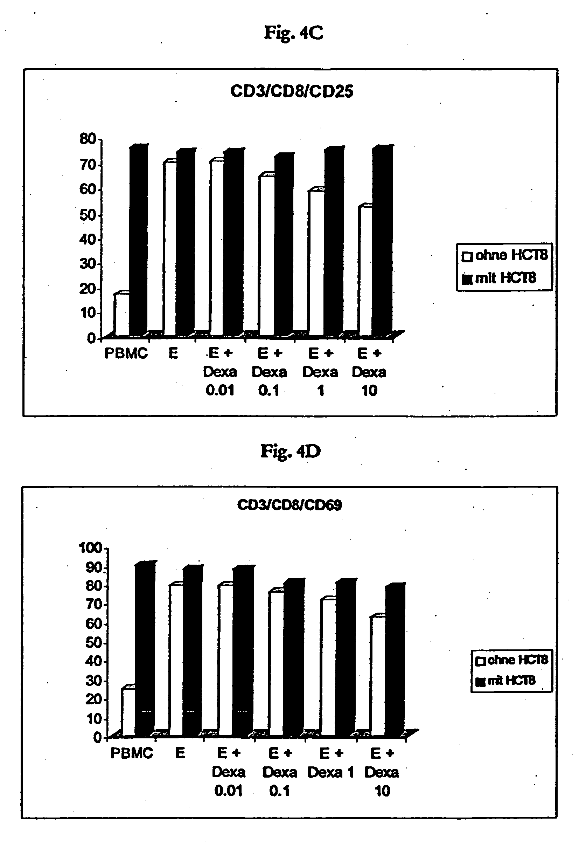

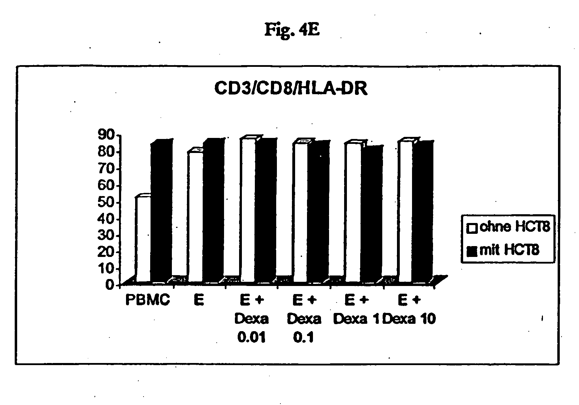

- Fig.4: zeigt graphische Darstellungen der Ergebnisse von Experimenten zur T-Zell-Aktivierung durch den trifunktionellen bispezifischen Antikörper mit der Spezifität anti-EpCAM x anti-CD3 (Konzentration von 10⁶ Zellen/ml) unter dem Einfluss der gleichzeitigen Gabe von Dexamethason (Konzentrationen von 0,01, 0,1, 1 und 10 µg/ml). In den Figuren 4A bis 4E ist jeweils auf der y-Achse der prozentuale Anteil der CD3/CD4 (T-Helferzellen) bzw. CD3/CD8 (T-Killerzellen)-positiven T-Lymphozyten mit Expression eines Aktivierungsmarkers (Fig. 4A und 4C: CD25; Fig. 4B und 4D: CD69; Fig. 4E: HLA-DR) aufgetragen. Die Messung erfolgte durch FACS-Analyse. Die jeweils linken Balken stellen die Ergebnisse der Stimulation ohne Kontakt zu HCT8-Zielzellen (Konzentration 5 x 10⁴ Zellen/ml) dar, während die jeweils rechten Balken die Messwerte bei Stimulation mit Kontakt zu HCT8-Zielzellen angeben. Bei Stimulation mit dem trifunktionellen bispezifischen Antikörper unter Kontakt zu den HCT8-Zielzellen, die der spezifischen Stimulation mit Bindung des Ziel-Antigens entspricht, wird die T-Zell-Aktivierung durch Dexamethason nicht beeinflusst, wie die unverändert hohen Messwerte in den Figuren 4A bis 4E belegen.

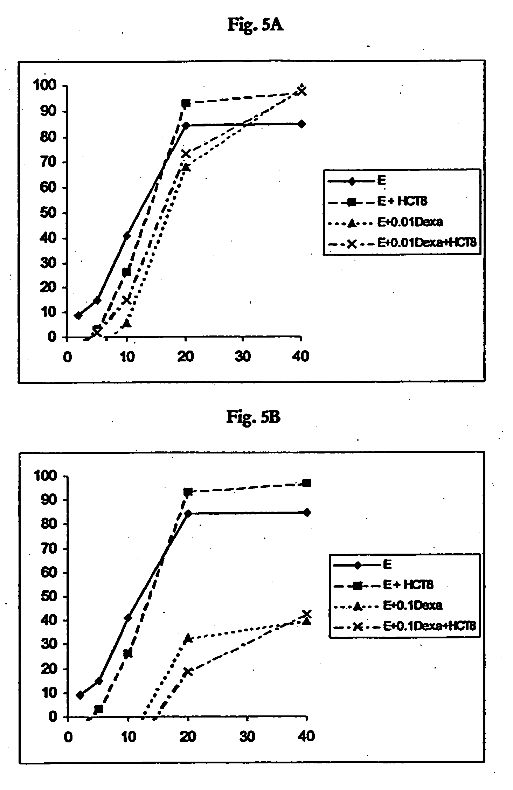

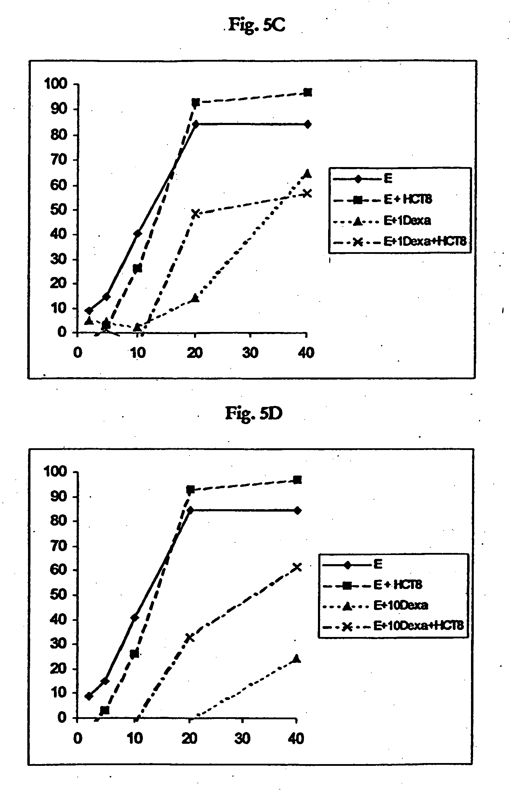

- Fig. 5.: stellt in Diagrammen die Ergebnisse der Messung der spezifischen prozentualen Zytotoxizität, die in einem zweistündigen Zytotoxizitätsassay mit Fluoreszenz-markierten HCT8-Tumorzellen bestimmt wurde. In den Figuren 5A bis 5D ist die spezifische prozentuale Zytotoxizität gegen HCT8 in Abhängigkeit vom Verhältnis Effektor-:Zielzellen (E/T) aufgetragen, wobei der Zytotoxizitätstest jeweils bei einem Verhältnis der Effektor- zu den Zielzellen von 40:1, 20:1, 10:1, 5:1 und 2,5:1 durchgeführt wurde. In jedem Experiment erfolgte eine Stimulation mit 100 µg/ml trifunktionellen bispezifischen Antikörpern mit der Spezifität anti-EpCAM x anti-CD3 (E) ohne HCT8-Zellen, in Gegenwart von HCT8-Zellen (E + HCT8) sowie unter zusätzlicher Gabe von Dexamethason in Konzentrationen von 0,01 µg/ml, 0,1 µg/ml, 1,0 µg/ml oder 10,0 µg/ml ohne (E + µg/ml Dexa) bzw. mit Kontakt zu HCT8-Zellen (E + µg/ml Dexa + HCT8).
- Fig. 5A: dokumentiert den Einfluss von Dexamethason in einer Konzentration von 0,01 mg/ml auf die spezifische prozentuale Zytotoxizität. Im Vergleich zu PBMC ohne Dexamethason ergibt sich hier keine Hemmung der spezifischen prozentualen Zytotoxizität durch das Glukokortikoid.
- Fig. 5B: stellt die Ergebnisse der entsprechenden Messungen hinsichtlich des Einflusses von 0,1 µg/ml Dexamethason dar. Bei dieser Dexamethason-Konzentration ergibt sich eine deutliche Hemmung der spezifischen prozentualen Zytotoxizität um etwa 50 Prozentpunkte. Dabei ergab sich kein nennenswerter Unterschied, wenn die Stimulation in Gegenwart oder in Abwesenheit von HCT8-Zellen durchgeführt wurde.
- Fig. 5C: zeigt die Ergebnisse der Vergleichsmessungen mit Dexamethason in einer Konzentration von 1 µg/ml. Auch hier ergab sich eine deutliche Hemmung der spezifischen prozentualen Zytotoxizität, die in etwa das gleiche Niveau zeigte, wie es bei einer Glukokortikoid-Konzentration von 0,1 µg/ml festgestellt wurde (vgl. Fig. 5B), wenn die Stimulation der PBMC in Anwesenheit von HCT8-Zellen erfolgte. Bei einem Verhältnis der Effektor-:Zielzellen von 20:1 ergab sich bei Abwesenheit von HCT8 eine deutlich stärkere Hemmung der prozentualen spezifischen Zytotoxizität der PBMC.
- Fig. 5D: gibt die entsprechenden Messungen bei einer Dexamethason-Konzentration von 10 µg/ml wieder. Bei Stimulation der PBMC in Gegenwart von HCT8-Zellen ergab sich bei dieser Dexamethason-Konzentration eine prozentuale spezifische Zytotoxizität, die in etwa derjenigen entsprach, die bei Dexamethason-Konzentrationen von 0,1 und 1 µg/ml festgestellt wurde (vgl. Fig. 5B und 5C): Bei einem Verhältnis der Effektor-:Zielzellen von 20:1 ergab sich demgemäß eine prozentuale Zytotoxizität von 30%, während bei einem doppelt so großen Verhältnis von Effektor-:Zielzellen die spezifische prozentuale Zytotoxizität bei etwa 60% lag. Im Gegensatz dazu fielen bei einer Stimulation der PBMC in Abwesenheit von HCT8-Zellen die Zytotoxizitätswerte gegenüber den geringeren Dexamethason-Konzentrationen weiter ab.

Gemäß den in den Figuren 5A bis 5D dargestellten Ergebnissen hat Dexamethason auf die Zytotoxizität von stimulierten PBMC bei einer Konzentration von 0,01 µg/ml keine Auswirkung. Ab einer Konzentration von 0,1 µg/ml ergibt sich eine deutliche Verminderung der Zytotoxizität unabhängig von Abwesenheit oder Gegenwart von HCT8 während der Stimulation. Bei einer Stimulation in Gegenwart von HCT8 bleibt die prozentuale spezifische Zytotoxizität auf einem Niveau von 40% bis 60% der Zytotoxizität, die ohne Dexamethason gemessen wurde, wenn Dexamethason über einen Konzentrations-Bereich von 3 Größenordnungen (0,1 bis 10 µg/ml) bei einem Verhältnis der Effektor-:Zielzellen von 40:1 gegeben wurde. Daher bleibt bei einem entsprechenden Effektor-(E):Zielzellen(T)-Verhältnis bei Zugabe von Glukokortikoid in einem großen Konzentrationsbereich eine deutliche prozentuale Zytotoxizität der in Gegenwart von HCT8-Zellen stimulierten PBMC konstant erhalten. Im Gegensatz dazu fällt die Zytotoxizität abhängig von der Dexamethason-Konzentration nach Stimulation der PBMC ohne HCT8 weiter deutlich ab.

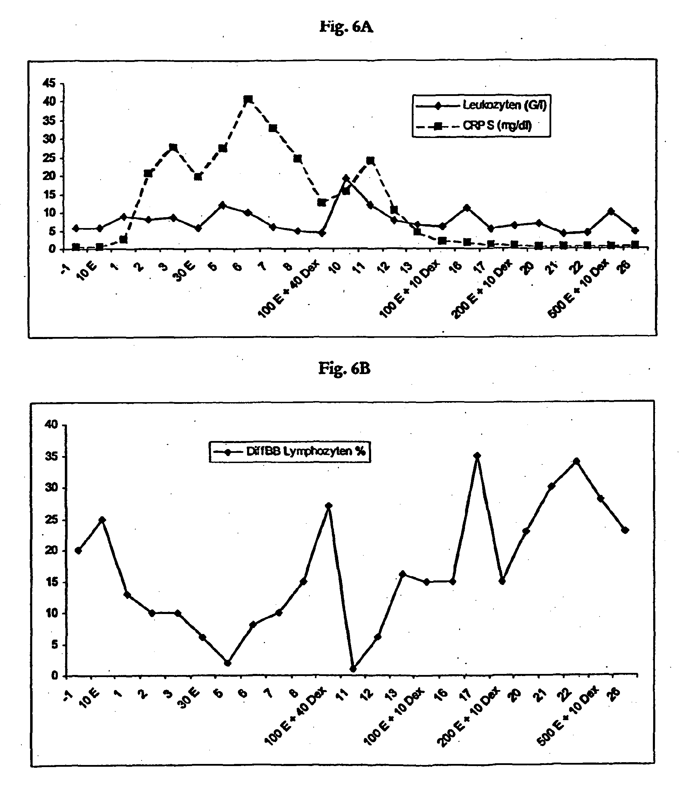

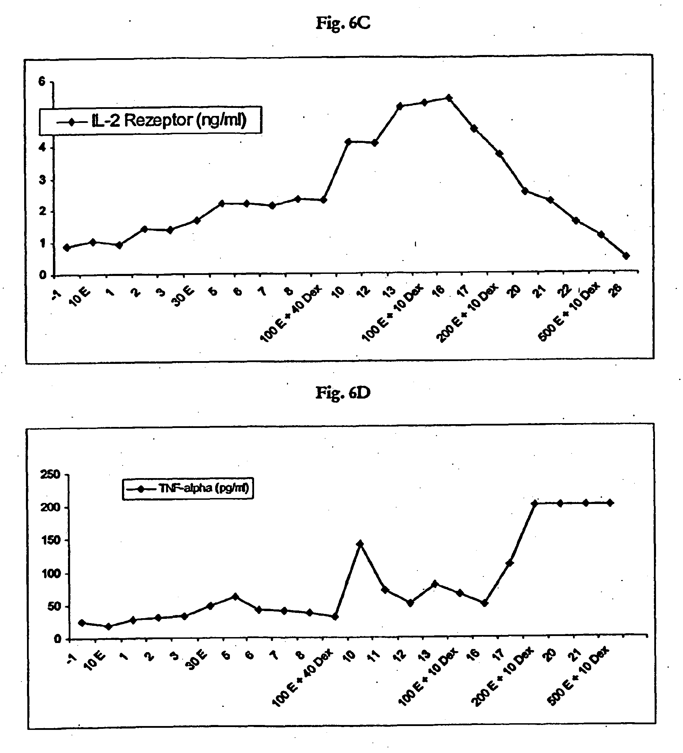

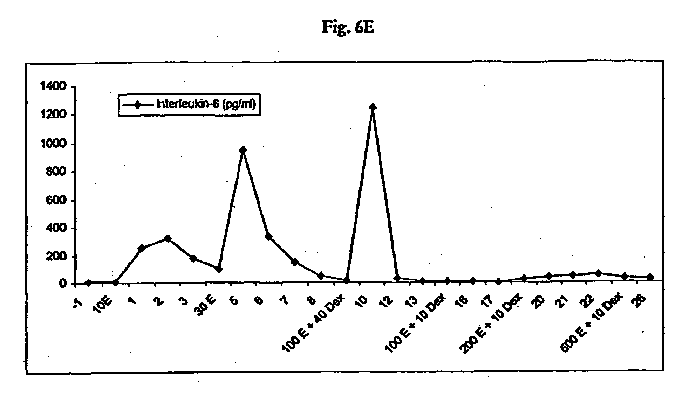

- Fig. 6: dokumentiert die Laborwerte immunologischer Parameter eines Patienten mit einem Chemotherapie-resistenten Magenkarzinom, der erfindungsgemäß mit einem trifunktionellen, bispezifischen Antikörper (anti-EpCAM x anti-CD3) zur Immunstimulation gegen die Tumorzellen sowie zur Inhibition der mit der Immuntherapie zusammenhängenden Nebenwirkungen mit Glukokortikoid (Dexamathason) behandelt wurde. Auf der y-Achse der Figuren 6A bis 6E sind jeweils die Messwerte in der jeweils angegebenen Einheit aufgetragen. Auf der x-Achse ist der Therapie-Verlauf in Tagen dargestellt. E bedeutet dabei eine Gabe des vorstehend genannten immunstimulatorischen Antikörpers mit der Dosierung i.p. in µg und Dex bedeutet die Dexamethason-Dosis in mg, die i.v. verabreicht wurde.
- Fig. 6A: stellt den Verlauf der Leukozyten-Konzentration (in G/l) (◆) sowie die Serum-CRP-Konzentrationen (in mg/dl) (■) dar. Die ersten beiden Antikörper-Gaben am Tag 0 sowie Tag 4 erfolgten ohne zusätzliche Verabreichung des Glukokortikoids. Unter dieser Therapie steigt insbesondere die Serumkonzentration des "Akute Phase Proteins" CRP bis auf 40 mg/dl an. Im weiteren Verlauf der Therapie mit zusätzlicher Applikation von Dexamethason bei gleichzeitig deutlicher Erhöhung der Antikörper-Dosierung auf bis zu 500 µg fällt der CRP-Serumspiegel wieder auf normale Werte ab. Auch die Leukozyten-Konzentration bleibt unter der erfindungsgemäßen Kombinations-Therapie unter 20 G/l. Normalwerte bei gesunden Erwachsenen schwanken zwischen 4 und 11 G/L Die beobachtete Erhöhung der Leukozytenzahl ist auch Ausdruck der erwünschten Immunstimulation.
- Fig. 6B: stellt den Verlauf des prozentualen Anteils der Lymphozyten im Differentialblutbild dar. Die beobachteten Schwankungen sowohl unter der Antikörper-Monotherapie zu Beginn der Behandlung als auch bei zusätzlicher Gabe von Dexamethason unter gleichzeitiger starker Erhöhung der Dosis des immuntherapeutischen Antikörpers entsprechen den Erwartungen. Insbeondere zum Ende der Verabreichung der erfindungsgemäßen Kombination von Antikörper und Glukokortikoid liegen die gemessenen Werte in etwa im Normalbereich eines gesunden Erwachsenen (20 bis 30% Lymphozyten).
- Fig. 6C: zeigt den Verlauf der Serumkonzentration des IL-2-Rezeptors. Die Werte steigen von etwa 1 bis etwa 2 ng/ml zu Beginn der Behandlung zunächst bei Erhöhung der Dosis des immunstimulatorischen Antikörpers auf 100 µg i.p. und zusätzlicher Gabe von Dexamethason bis auf etwa 5,5 ng/ml an, sinken jedoch trotz weiterer deutlicher Dosiserhöhung des trifunktionellen, bispezifischen Antikörpers und gleichzeitiger Verabreichung des Glukokortikoids bei einer Dosis von 10 mg i.v. wieder unter 1 ng IL-2-Rezeptor/ml zum Ende der Behandlung ab.
- Fig. 6D: zeigt den Verlauf der TNF-α-Konzentration im Serum (in pg/ml), die bei dem Patienten mit dem Magenkarzinom gemessen wurde. TNF-α steigt unter der Therapie mit einer Dosissteigerung von 10 µg bis auf 500 µg des immuntherapeutischen Antikörpers als Ausdruck einer zellulären Immunaktivität von einem Wert von unter 50 pg/ml bei niegrig dosierter Antikörpermonotherapie auf etwa 200 pg/ml bei einer Gabe von 200 bzw. 500 µg i.p. des bispezifischen Antikörpers plus jeweils 10 mg i.v. Dexamethason an. Somit wird aufgrund der die Nebenwirkungen unterdrückenden Gabe des Glukokortikoids, die eine erhebliche Dosissteigerung des immunstimulatotischen Antikörpers ermöglicht, eine hervorragende zelluläre spezifische Immunaktivität bei der erfindungsgemäßen Therapie bzw. Verwendung des Glukokortikoids beobachtet.
- Fig. 6E: zeigt den Verlauf der IL-6-Konzentration im Serum (in pg/ml) des Patienten mit Magenkarzinom unter der auf der x-Achse aufgetragenen Therapie. Nach der ersten und zweiten Verabreichung des immunstimulierenden Antikörpers ohne gleichzeitige Gabe von Dexamethason (am Tag 0 und Tag 4) sind deutliche Erhöhungen der IL-6-Konzentration auf etwa 300 bzw. knapp 1000 pg/ml zu verzeichnen. Auch nach der ersten Kombinationsgabe von Antikörper und Glukokortikoid (100 µg Antikörper i.p. plus 40 mg Dexamethason i.v. am Tag 9) ist ein kurzer Anstieg der IL-6-Konzentration mit einem Maximalwert von etwa 1200 pg/ml zu beobachten. Die IL-Konzentration erreicht jedoch sehr schnell wieder Basiswerte und bleibt trotz Erhöhung der Antikörper-Dosis auf bis zu 500 µg i.p. unter gleichzeitiger Verabreichung von 10 mg Dexamethason i.v. konstant bei niedrigen Werten.

Die bei einem Patienten mit Magenkarzinom festgestellten Werte der immunologischen Parameter zeigen, dass unter der Kombinationstherapie mit trifunktionellem bispezifischen Antikörper der Spezifität anti-EpCAM x anti-CD3 plus Dexamethason eine deutliche zelluläre spezifische Immunaktivität ausgelöst wird, wie der Anstieg der TNF-α-Konzentration zeigt. Alle anderen Parameter bleiben unter der immensen Dosissteigerung des Antikörpers aufgrund der Verabreichung des Glukokortikoids weitgehend konstant. Es ist hierbei zu beachten, dass außer TNF-α keiner der gemessenen immunologischen Parameter die Antikörpervermittelte Wirkung auf die Tumorzelle widerspiegelt, sondern der immunologischen Nebenwirkung entspricht. Insbesondere zeigt sich trotz der starken Dosissteigerung des Antikörpers unter gleichzeitiger Applikation von Dexamethason kein Anzeichen eines SIRS (engl. "systemic inflammatory response syndrome"). SIRS ist ein Entzündungssyndrom, das aufgrund einer starken systemischen Zytokin-Freisetzung bei Antikörper-Therapien, insbesondere immunstimulatorischen Therapien, in hoher Dosis auftritt und mit einem Organversagen bis hin zu einem letalen Ausgang einhergehen kann. Die unter der erfindungsgemäßen Kombinationstherapie beobachteten immunologischen Parameter bei einem Patienten mit einem Chemotherapie-resistenten Magenkarzinom zeigen für eine derartige starke Nebenwirkung überraschenderweise keinerlei Anzeichen.

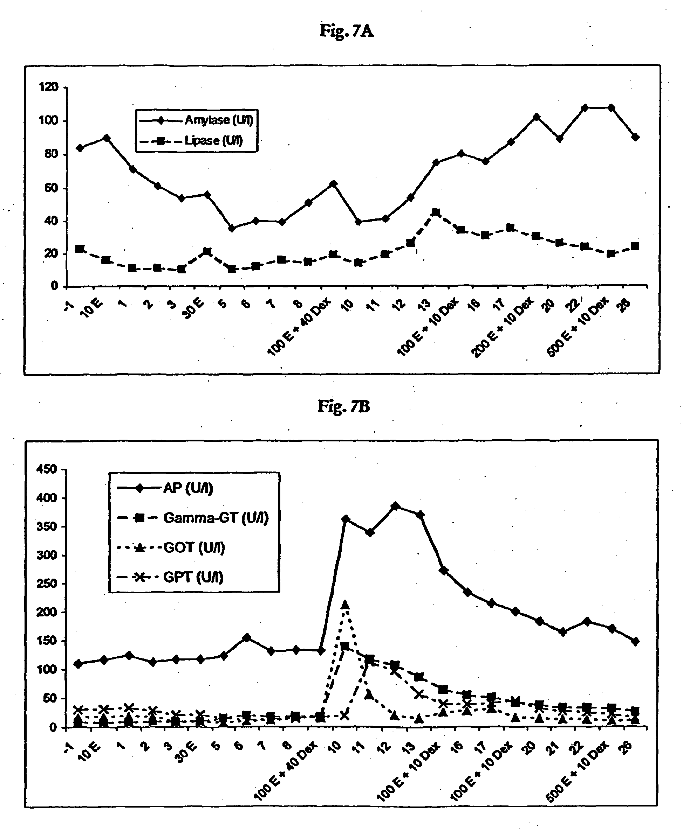

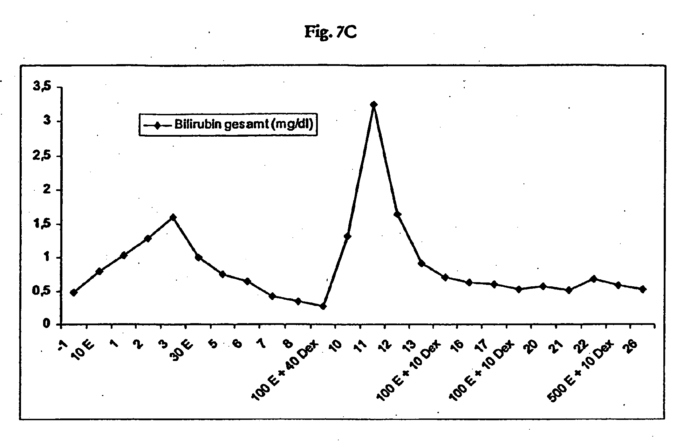

- Fig. 7: zeigt Diagramme, welche die Nebenwirkungen der vorstehend dargelegten Therapie eines Patienten mit Chemotherapie-resistentem Magenkarzinom auf die Pankreas- und Leberwerte darstellen.
- Fig. 7A: zeigt den Serumkonzentrationsverlauf der Pankreas-spezifischen Enzyme α-Amylase (◆) und Lipase (■) jeweils in U/L Bei keinem Pankreas-Parameter kommt es zu einer abnormen Erhöhung als Zeichen einer unerwünschten Nebenwirkung trotz starker Dosissteigerung des immunstimulatorischen Antikörpers bei der erfindungsgemäßen Kombinationsbehandlung. (Normalwerte eines gesunden Erwachsenen: α-Amylase < 120 U/l; Lipase < 190 U/l)
- Fig. 7B: zeigt den entsprechenden Verlauf der Serumkonzentrationen der Leber-spezifischen Enzyme alkalische Phosphatase (AP; ◆), γ-Glutamyltransferase (γ-GT; ■), Glutamat-Oxalacetat-Transaminase (GOT; ▲) und Glutamat-Pyruvat-Transaminase (GPT; x) (jeweils in U/l) unter der vorstehend beschriebenen Therapie eines Patienten mit Magenkarzinom. Die Referenzbereiche der Serumkonzentrationen der gemessenen Enzyme betragen: AP: 40 bis 190 U/l; γ-GT: 4 bis 18 U/l; GOT: 5 bis 15 U/l; GPT: 5 bis 19 U/L Lediglich zu Beginn der Antikörper-Glukokortikoid-Kombinationstherapie (Therapietage 10 bis 13) tritt eine Erhöhung der Serumkonzentrationen der Leberenzyme, insbesondere der AP, auf, die jedoch innerhalb kurzer Zeit wieder auf die Normalwerte zurückgehen. Somit kommt es auch bei den Konzentrationen der Leber-spezifischen Enzyme zu keiner der Dosissteigerung des immunstimulierenden Antikörpers entsprechenden Erhöhung als Zeichen eine unerwünschten Nebenwirkung unter gleichzeitiger Gabe von Dexamethason.
- Fig. 7C: stellt in einem Diagramm die Gesamtkonzentration an Bilirubin (in mg/dl) als weiterem Leberparameter in Abhängigkeit des Therapieverlaufs des Patienten mit Magenkarzinom dar. Abgesehen von einer schnell vorübergehenden Bilirubin-Erhöhung nach der Dosissteigerung des immunstimulierenden Antikörpers von 30 auf 100 µg liegt auch dieser Leberparameter bei einer weiteren Dosissteigerung im Refemezbereich von unter 1 mg/dL Daher wird trotz sehr hoher Dosierung des bispezifischen Antikörpers bei Verabreichung von Dexamehason keine Bilirubin-Erhöhung festgestellt, die eine unerwünschte Nebenwirkung anzeigen würde. In diesem Zusammenhang wurde bedeutsamerweise festgestellt, dass der Patient unter der Therapie mit gleichzeitiger Gabe von Dexamethason und immunstimulierendem Antikörper keinerlei schwerwiegende klinische Nebenwirkungen zeigte (vgL nachstehendes Ausführungsbeispiel 4).

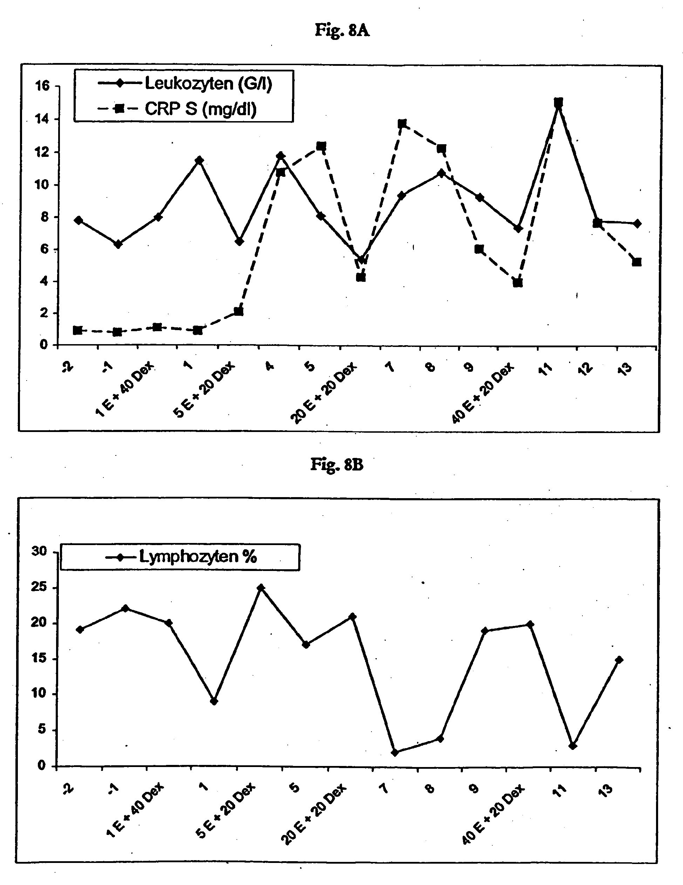

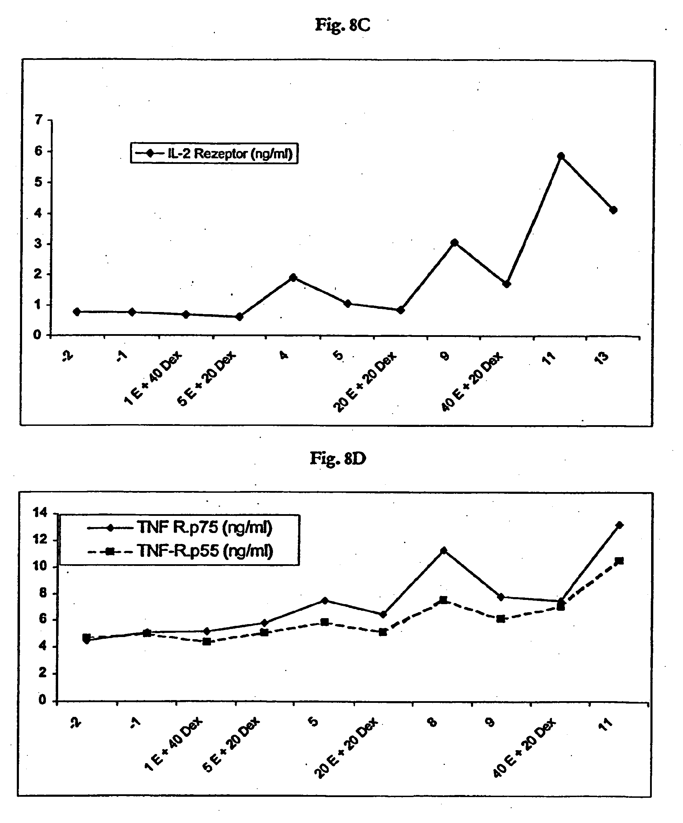

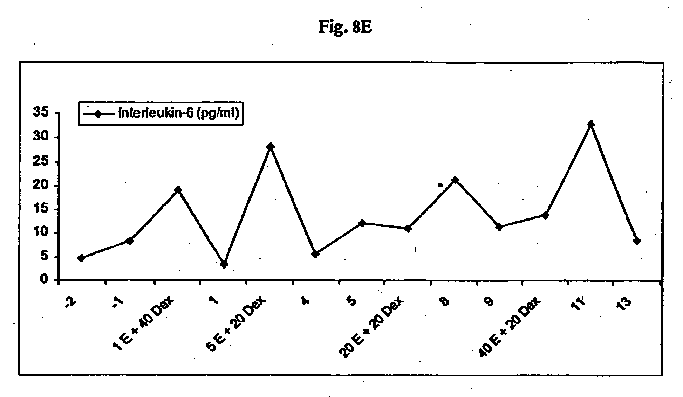

Es wurde ein weiterer Patient erfindungsgemäß mit einem Antikörper (trifunktioneller bispezifischer Antikörper mit der Spezifität anti-EpCAM x anti-CD3) und Dexamethason in immuntherapiert. Es handelte sich um einen weiblichen Patienten mit einem Adenokarzinom und diffuser Lebermetastasierung. Die Applikation des Antikörpers erfolgte über einen selektiven Katheter in die A. hepatica dextra. In der Fig. 8 sind wiederum Diagramme gezeigt, welche den Verlauf immunologischer Parameter in Abhängigkeit von der Therapie dokumentieren. Die graphische Darstellung der Parameter erfolgte wie vorstehend bei der Fig. 6 dargelegt.
- Fig. 8A: zeigt den Verlauf der der Leukozytenzahl und der CRP-Serumkonzentration, Fig. 8B stellt den prozentualen Anteil der Lymphozyten im Diffetentialblutbild in Abhängigkeit vom Therapieverlauf dar, Fig. 8C gibt den Verlauf der Konzentration des IL-2-Rezeptors wieder, Fig. 8D ist eine entsprechende Auftragung für den TNF-Rezeptor p75 und den TNF-Rezeptor p55, und Fig. 8E schließlich zeigt den Verlauf der IL-6-Konzentration unter der erfindungsgemäßen Kombinationstherapie. Unter der Therapie kam es trotz der Gabe von Dexamethason zu einem systemischen Anstieg des löslichen IL-2-Rezeptors (Fig. 8C) und der TNF-Rezeptoren p55 und p75 (Fig. 8D), was die erwünschte Immunaktivierung gegen Tumorzellen widerspiegelt. Gleichzeitig kam es unter einer Dosissteigerung von 1 µg Antikörper auf 40 µg Antikörper, d.h. bei einer Steigerung um den Faktor 40, zu keiner wesentlichen Erhöhung der Leukozyten, des Serum-CRP, des prozentualen Anteils der Lymphozyten und zu keiner signifikant erhöhten systemischen Freisetzung von IL-6 (vgl. Fig. 8A, 8B und 8E).

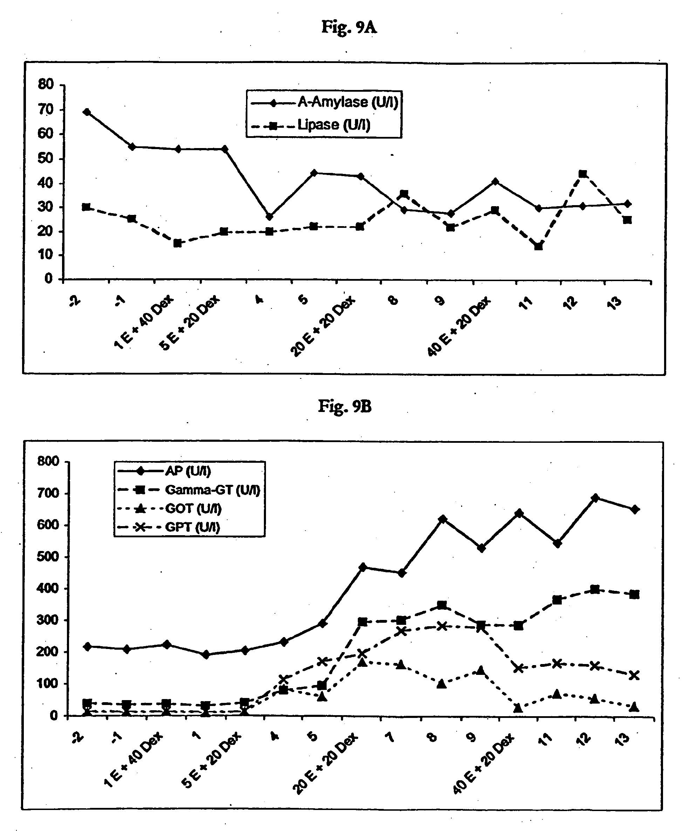

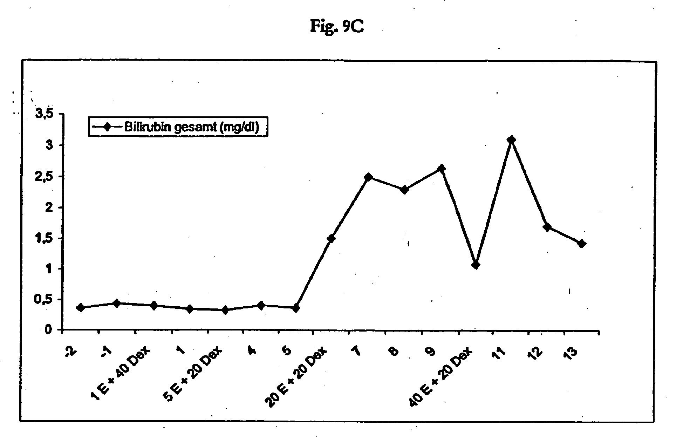

Auch bei der unter einem Adenokarzinom mit diffuser Lebermetastasierung leidenden Patientin wurden während der Therapie mit erfindungsgemäßer Verwendung des Glukokortikoids zusammen mit dem vorstehenden immunstimulierenden Antikörper Pankreas- und Leberspezifische Laborwerte bestimmt. Die Ergebnisse dieser Untersuchungen sind in Fig. 9 zusammengestellt. Die Auftragung erfolgte dabei wie vorstehend bei Fig. 7 beim Patienten mit Magenkrebs angegeben.
- Fig. 9A: zeigt wiederum die Serumkonzentrationen der Pankreas-spezifischen Enzyme α-Amylase und Lipase (jeweils in U/l) in Abhängigkeit von der auf der x-Achse dargestellten Therapie. Die Pankreaswerte lagen während der gesamten Therapie im Referenzbereich.
- Fig. 9B: zeigt eine entsprechende Auftragung der Leber-spezifischen Enzyme AP, γ-GT, GOT und GPT (ebenfalls in U/l). Zwar ist ein Anstieg der AP bei einer Steigerung der Antikörper-Dosis von 5 µg auf 40 µg ausgehend von 200 U/l auf etwa 700 U/l zu verzeichnen. Ebenso steigen die Konzentrationen von γ-GT und GPT unter dieser Dosissteigerung von deutlich unter 100 auf knapp 400 U/l bzw. 150 U/l an. Dieser Anstieg der Konzentrationen der Leber-spezifischen Enzyme weist jedoch nicht auf beunruhigende Nebenwirkungen hin, da die Erhöhung nach Abschluß der Therapie in vollem Umfang reversibel war und bei der Patientin zu keinem Zeitpunkt klinische Probleme auftraten. Dies gilt im übrigen auch für den Verlauf der Gesamt-Bilirubinkonzentration, die in Abhängigkeit vom Therapieverlauf in Fig. 9C dargestellt ist und einen weiteren Leber-spezifischen Laborparameter darstellt. Der Anstieg der Gesamt-Bilirubin-Konzentration von 0,5 mg/dl bei 5 µg Antikörper-Dosis auf bis zu 3 bis 3,5 mg/dl bei einer Antikörper-Dosis von 40 µg zeigt keine signifikante Nebenwirkung der Therapie auf die Leber der Patientin an. In diesem Zusammenhang ist ebenfalls zu beachten, dass die Leberwerte der Patientin, insbesondere die Leber-spezifischen Enzyme, aufgrund der bestehenden Lebermetastasen erhöht waren.

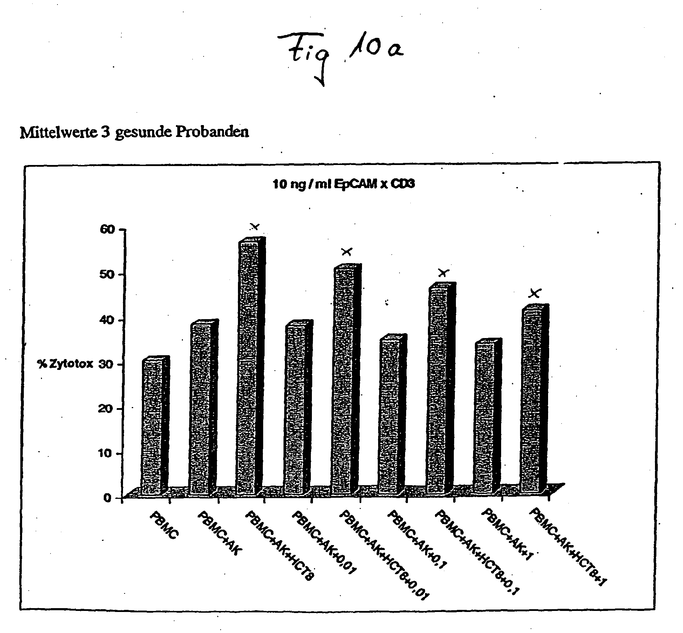

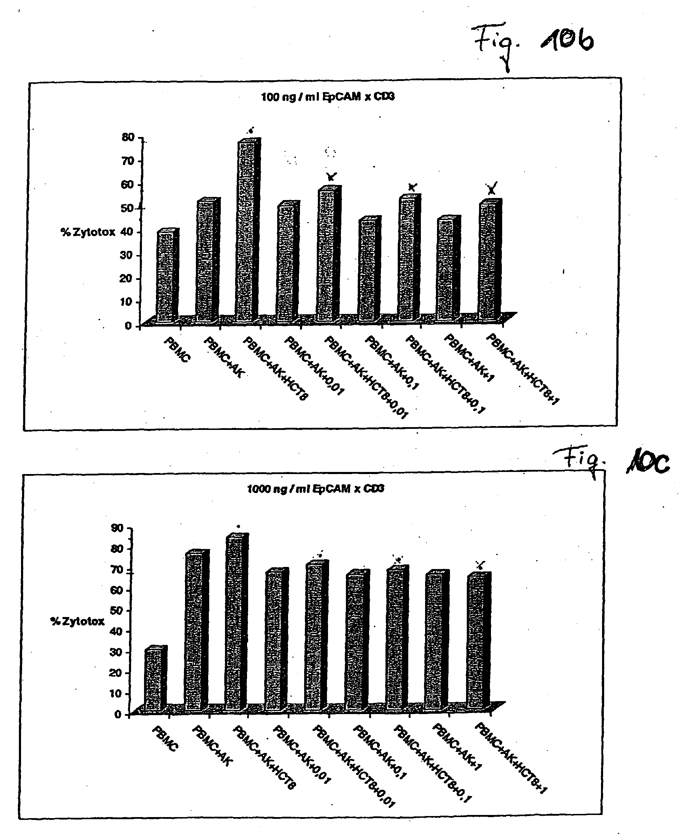

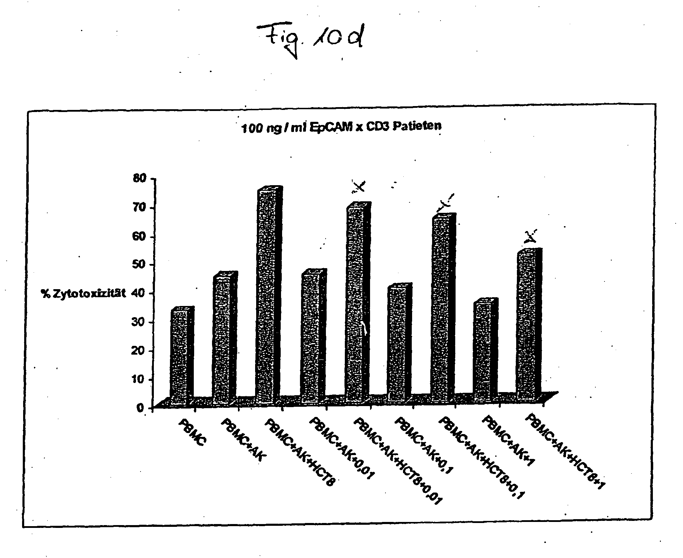

- Fig. 10: zeigt graphische Darstellungen von Zytotoxitätsassays, in denen die Ergebnisse der Messungen der spezifischen prozentualen Zytotoxität unter Verwendung von Fluoreszenz-markierten HCT8-Tumorzellen bestimmt wurden. Die Figuren 10A bis 10C stellen die Mittelwerte der an drei gesunden Probanden durchgeführten Messungen dar, während die Figur 10D die Mittelwerte der an sechs Patienten mit gastrointestimalen Karzinom durchgeführten Messungen datstellt. In den Experimenten erfolgte eine Stimulation mit trifunktionellen bispezifischen Antikörpern mit der Spezifität anti-EpCAM x anti-CD3 bei verschiedenen Konzentrationen von 10 ng/ml, 100 ng/ml und 1000 ng/ml. Die jeweilige Konzentration des verwendeten Antikörpers wird in dem Figuren 10A bis 10D angegeben. Die Konzentration der PBMC lag bei 10⁶ Zellen/ml, die der HCT8-Zellen bei 5 x 10⁴ Zellen/ml. In jedem der Experimente erfolgte die Stimulation mit dem Antikörper ohne HCT8-Zelen (PBMC+AK), in Gegenwart von HCT8-Zellen (PBMC+AK+HCT8) und unter zusätzlicher Verabreichung von Dexamethason in Konzentrationen von 0,01 µg/ml, 0,1µg/ml, 1,0 µg/ml (PBMC+AK+0,01 bzw. 0,1 bzw. 1). Auf der y-Achse ist die prozentuale Zytotoxizität aufgetragen, auf der x-Achse ist der jeweilige Versuchsansatz der PBMC aufgetragen, wobei unstimulierte PBMC als Kontrolle dienten (PBMC).
- Fig. 10A: zeigt die Ergebnisse der entsprechenden Messungen bei einer Antikörperkonzentration von 10 ng/ml. Bei stimulierten-PBMC mit Kontakt zu HCT8-Zielzellen und Gabe von Dexamethason ergibt sich eine Hemmung der spezifischen prozentualen Zytotoxizität um ungefähr 15 Prozentpunkte (von ca. 55 auf 41%), wobei die Hemmung mit steigender Dexamethason-Konzentration zunimmt. Die prozentuale Hemmung fällt bei Kontakt zu HCT8 deutlicher aus als in den Versuchsansätzen, die keinen Kontakt zu HCT8-Zellen haben. Hier erfolgt eine Hemmung lediglich um ca. 5 Prozentpunkte.
- Fig.10B: stellt die Ergebnisse der Messungen bei einer Antikörperkonzentration von 100 ng/ml dar. Bei stimulierten PBMC mit Kontakt zu HCT8-Zielzellen ergibt sich eine deutliche Hemmung der spezifischen prozentualen Zytotoxizität von ca. bis zu 30 Prozentpunkten (von ca. 78 auf 50%), wenn die zusätzliche Gabe von Dexamethason erfolgte, wobei die Hemmung mit steigender Dexamethason-Konzentration zunimmt. Im Gegensatz dazu ergibt sich nur eine geringfügige Hemmung der Zytotoxyzität um ungefähr 10 Prozentpunkte bei stimulierten PBCM ohne Kontakt zu HCT8 und Gabe von Dexamethason, die ebenfalls mit steigender Dexamethason-Konzentration zunimmt
- Fig. 10C: zeigt die Ergebnisse der entsprechenden Messungen bei einer Antikörperkonzentration von 1000 ng/ml. Es erfolgt eine Hemmung der Zytotoxizität durch Dexamethason, wobei diese jedoch insgesamt geringer ausfällt, als es bei einer Stimulation mit einer Antikörperkonzentration von 100ng/ml (Figur 10B) der Fall ist Bei stimulierten PBMC mit Kontakt zu HCT8-Zielzellen ergibt sich eine Hemmung der spezifischen prozentualen Zytotoxizität um ca. bis zu 20 Prozentpunkte (von ca. 85 auf 67%), wenn die zusätzliche Gabe von Dexamethason erfolgte, wobei die Hemmung mit steigender Dexamethason-Konzentration zunimmt. In den Versuchsansätzen stimulierter PBMC ohne Kontakt zu HCT8 und bei Gabe von Dexamethason, ergibt sich lediglich eine minimale Hemmung der Zytotoxyzität um ca. 2 Prozentpunkte, die wiederum mit steigender Dexamethason-Konzentration zunimmt.
- Fig. 10D: zeigt die entsprechenden Durchschnittsmessungen bei Tumorpatienten mit gastrointestinalen Tumoren. Diese Messungen wurden bei einer Antikörperkonzentration von 100 ng/ml durchgeführt Wie auch hier zu sehen ist, erfolgt eine deutliche Hemmung der Zytotoxizität um ungefähr 27 Prozentpunkte (von ca. 78% auf 51%) unter Gabe von Dexamethason. Die Hemmung fällt wiederum stärker aus, wenn eine Stimulation mit Kontakt zu HCT8-Zielzellen erfolgt Die Ergebnisse sind von der Antikörperkonzentration her mit denen aus Figur 10B (gesunde Probanden) vergleichbar. Aus einem solchen Vergleich ist festzustellen, dass sowohl bei gesunden Probenden als auch bei Tumorpatienten eine deutliche Hemmung der spezifischen prozentualen Zytotoxizität durch Verabreichung des Glukokortikoids Dexamethason erfolgt

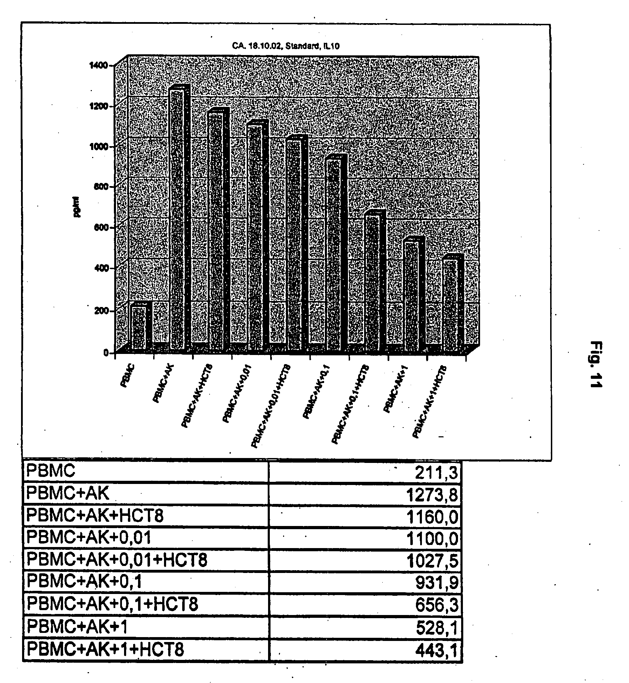

- Figuren 11 bis 13: zeigen graphische Darstellungen der Ergebnisse der Freisetzung von verschiedenen Zytokinen bei gesunden Probanden durch PBMC (bei einer Konzentration von 10⁶ Zellen/ml) nach Stimulation mit einem trifunktional bispezifischen Antikörper der Spezifität anti-EpCAM x anti-CD3 (AK) (bei einer Konzentration von 100 ng/ml) ohne bzw. in Gegenwart von EpCAM-positiven Tumorzellen (HCT8) (bei einer Konzentration von 5 x 10⁴ Zellen/ml). Auf den y-Achsen ist die Konzentration des jeweiligen Zytokins in pg/ml aufgetragen. Auf den x-Achsen sind die jeweiligen Stimulationsamsätze der PBMC aufgetragen. Mit "0,01", "0,1" bzw. "1" wird die jeweilige Konzentration des zugegebenen Dexamethasons in µg/ml angegeben. Die Werte von unstimulierten PBMC als Kontrolle sind ebenfalls angegeben ("PBMC").

Die Stimulation der PBMC ohne Kontakt mit EpCAM-positiven Tumorzellen (HCT8-Zellen) entspricht (wie auch in den Figuren 1 bis 3) der unspezifischen Zytokinfreisetzung, die unabhängig von der Antigen-Bindung (Antikörper-Antigen-Wechselwirkung) ist. Die Freisetzung der jeweiligen Zytokine erfolgt demnach systemisch durch die Gesamtheit der PBMC. Die Stimulation der PBMC mit Kontakt zu EpCAM-positiven Tumorzellen entspricht dagegen der Freisetzung der jeweiligen Zytokine direkt am Ort der Antigen-Bindung durch die PBMC. Die in diesen Versuchsansätzen gemessenen jeweiligen Zytokinfreisetzungen entsprechen daher der spezifischen (zielgerichteten) Wirkung des verwendeten Antikörpers und stellen somit keine unerwünschte Nebenwirkung (systemische Freisetzung) dar. Die jeweiligen Zytokine werden dann nur von den direkt an der spezifischen Immunreaktion beteiligten Immunzellen freigesetzt
- Fig. 11: zeigt die Ergebnisse der Freisetzung von Interleukin 10 (IL-10). Bei IL-10 handelt es sich um ein immunsuppressives Zytokin. Es ist zu erkennen, dass die Freisetzung von IL-10 bei Stimulation mit dem Antikörper und Gabe von Dexamethason abnimmt. Diese Abnahme verstärkt sich mit steigender Konzentration von Dexamethason.

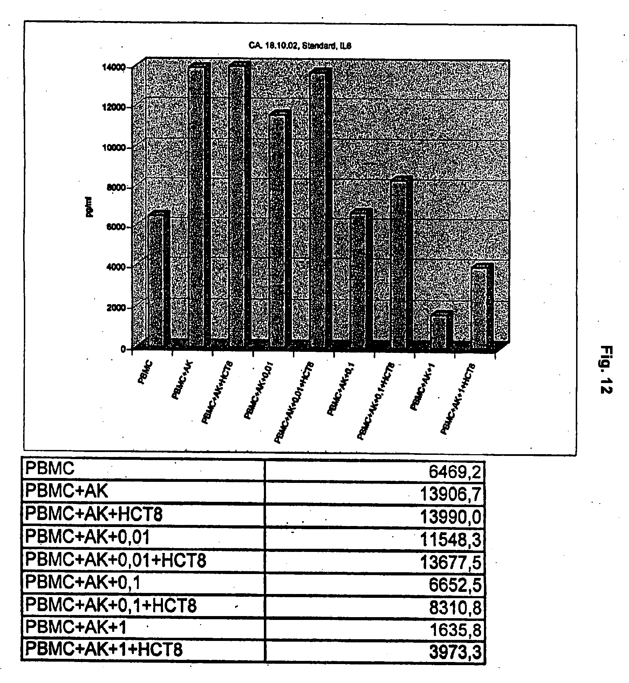

- Fig. 12: zeigt die Ergebnisse der Freisetzung von Interleukin 6 (IL-6). Die Freisetzung des proinflammatorischen Zytokins IL-6 nimmt bei Stimulation mit dem Antikörper und Gabe von Dexamethason ab. Diese Abnahme verstärkt sich mit steigender Konzentration von Dexamethason. Die Freisetzung von IL-6 nimmt weiterhin deutlich stärker ab, wenn kein gleichzeitiger Kontakt mit HCT8 vorliegt Die absolut höhere Freisetzung des Zytokins bei Kontakt mit HCT8-Zellen ist hierbei nicht als Zeichen einer erhöhten Nebenwirkung (systemische Zytokinfreisetzung) zu werten, sondern erklärt sich aus der spezifischen immunologischen Wirkung am Ort der Antigen-Bindung. D.h., ohne Kontakt zu den HCT8-Zellen (Nebenwirkung) nimmt die Freisetzung von IL-6 erfindungsgemäß schneller ab, als mit Kontakt zu HCT8-Zellen (spezifische Wirkung). Anders ausgedrückt, liegt eine hohe spezifische Wirkung (Freisetzung des Zytokins am Ort der Antigen-Bindung) vor.

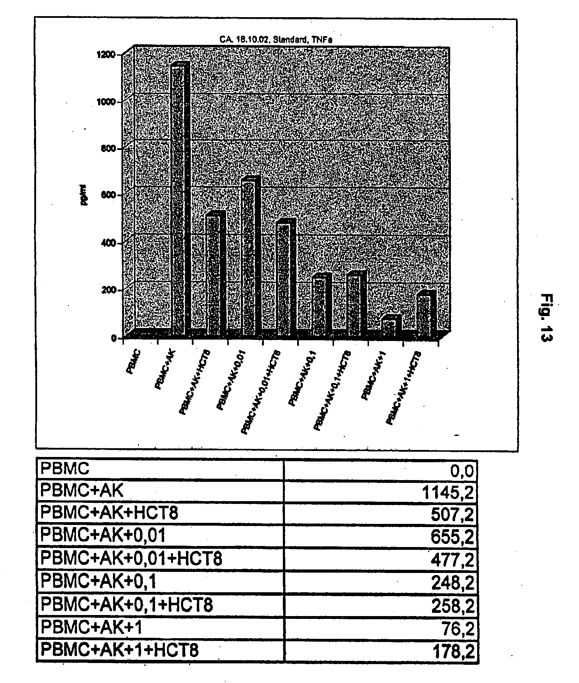

- Fig. 13: zeigt die Ergebnisse der Freisetzung von TNF-α. TNF-α ist, wie IFN-γ, ein Zytokin der zellulären Immunität. Die Freisetzung von TNF-α nimmt bei Stimulation mit dem Antikörper und Gabe von Dexamethason ab. Diese Abnahme verstärkt sich mit steigender Konzentration von Dexamethason. Ab einer Konzentration von 0,1 µg/ml Dexamethason ohne gleichzeitigen Kontakt zu HCT8 (systemische Freisetzung; Nebenwirkung) nimmt die Freisetzung von TNF-α stärker ab, als bei Kontakt mit HCT8 (spezifische Wirkung). Die absolut höhere Freisetzung von TNF-α bei Kontakt mit HCT8 stellt auch hier kein Zeichen einer erhöhten Nebenwirkung (systemische Zytokinfreisetzung) dar, sondern erklärt sich aus der spezifischen immunologischen Wirkung am Ort der Antigen-Bindung., d.h., die Nebenwirkung nimmt erfindungsgemäß stärker ab als die spezifische Wirkung.

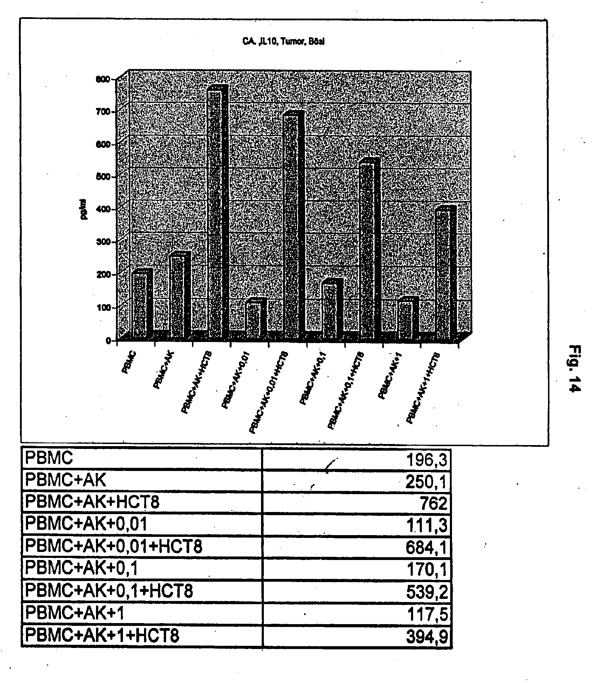

- Fig. 14 bis 16: zeigen graphische Darstellungen der Ergebnisse der Freisetzung von verschiedenen Zytokinen bei Tumorpatienten durch PBMC. Die Versuchsansätze (Konzentrationen, verwendete Substanzen bzw. Zellen) entsprechen denen der in Figuren 11 bis 13 dargestellten Ansätze. Ebenfalls wurde die Freisetzung der gleichen Zytokine gemessen. Dadurch können die Ergebnisse der gesunden Probanden (Figuren 11 bis 13) mit denen der Tumorpatienten (Figuren 14 bis 16) verglichen werden. Ebenfalls entspricht die Auftragung auf den y- und x-Achsen der Graphiken denen der Figuren 11 bis 13. Auch hier entspricht die Stimulation der PBMC ohne Kontakt zu HCT8 (gemäß Figuren 1 bis 3; 11 bis 13) der unspezifischen Zytokinfreisetzung, die unabhängig von der Antigen-Bindung ist. Die Freisetzung der jeweiligen Zytokine erfolgt dann systemisch durch die Gesamtheit der PBMC. Die Stimulation der PBMC mit Kontakt zu HCT8 entspricht der Freisetzung der jeweiligen Zytokine direkt am Ort der Antigen-Bindung durch die PBMC. Die in den letztgenannten Versuchsansätzen gemessenen jeweiligen Zytokinfreisetzungen entsprechen daher der spezifischen (zielgerichteten) Wirkung des verwendeten Antikörpers und stellen keine unerwünschte Nebenwirkung (systemische Freisetzung) dar. Die jeweiligen Zytokine werden in diesem Fall nur von den direkt an der spezifischen Immunreaktion beteiligten Immunzellen freigesetzt
- Fig. 14: zeigt die Ergebnisse der Freisetzung von Interleukin 10 (IL-10). Die Freisetzung von IL-10 nimmt bei Stimulation mit dem Antikörper und Gabe von Dexamethason ab. Diese Abnahme verstärkt sich mit steigender Konzentration von Dexamethason. Die Freisetzung von IL-10 nimmt schwächer ab, wenn kein gleichzeitiger Kontakt mit HCT8 vorliegt Die absolut höhere Freisetzung des Zytokins bei Kontakt mit HCT8-Zellen ist hierbei nicht als Zeichen einer erhöhten Nebenwirkung (systemische Zytokinfreisetzung) zu werten, sondern resultiert aus der spezifischen immunologischen Wirkung am Ort der Antigen-Bindung. D.h., ohne Kontakt zu HCT8 (Nebenwirkung) nimmt die Freisetzung von IL-10 mit steigender Dexamethason-Konzentration erfindungsgemäß schneller ab, als mit Kontakt zu HCT8 (spezifische Wirkung). D.h. weiterhin, dass wunschgemäß eine sehr starke spezifische Wirkung (Freisetzung des Zytokins am Ort der Antigen-Bindung) vorliegt.

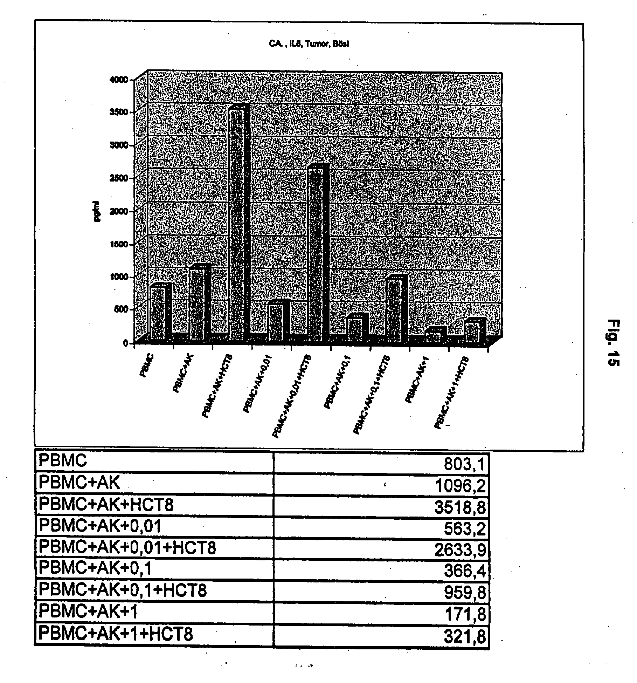

- Fig. 15: zeigt die Ergebnisse der Freisetzung von Interleukin 6 (IL-6). Auch hier nimmt, wie in Figur 14, die Freisetzung von IL-6 bei Stimulation mit dem Antikörper und Gabe von Dexamethason ab, wobei sich auch hier die Abnahme mit steigender Konzentration von Dexamethason verstärkt. Die Freisetzung von IL-6 nimmt drastisch stärker ab, wenn kein gleichzeitiger Kontakt mit HCT8 vorliegt. Die absolut höhere Freisetzung des Zytokins bei Kontakt mit HCT8-Zellen ist auch hier nicht als Zeichen einer erhöhten Nebenwirkung (systemische Zytokinfreisetzung) zu werten, sondern resultiert aus der spezifischen immunologischen Wirkung am Ort der Antigen-Bindung. D.h., ohne Kontakt zu HCT8 (Nebenwirkung) nimmt die Freisetzung von IL-6 erfindungsgemäß mit steigender Dexamethason-Konzentration weitaus stärker ab, als mit Kontakt zu HCT8 (spezifische Wirkung). Demnach bleibt festzustellen, dass eine sehr starke spezifische Wirkung (Freisetzung des Zytokins am Ort der Antigen-Bindung) vorliegt.

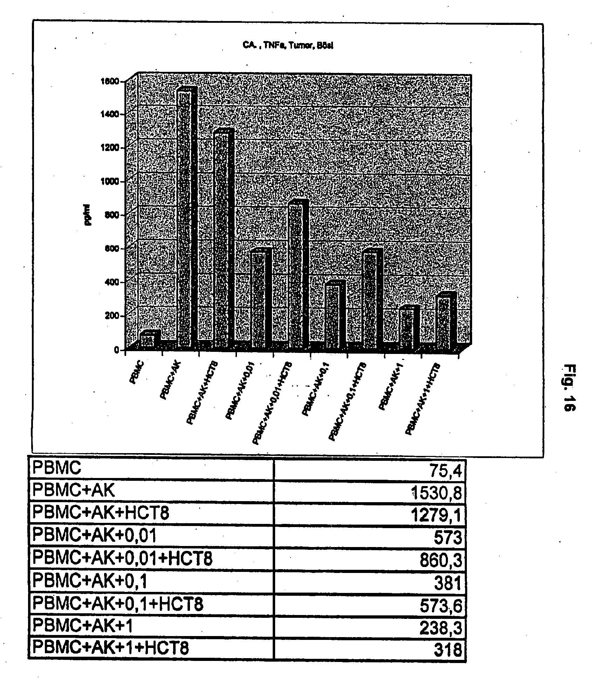

- Fig. 16: zeigt die Ergebnisse der Freisetzung von TNF-α. Bei Stimulation mit dem Antikörper und Gabe von Dexamethason nimmt die TNF-α-Freisetzung ab, und zwar umso stärker, je höher die Konzentration von Dexamethason ist. Die Freisetzung von TNF-α nimmt auch hier erwartungsgemäß stärker ab, wenn kein gleichzeitiger Kontakt mit HCT8 vorliegt, wobei auch hier die absolut höhere Freisetzung des Zytokins bei Kontakt mit HCT8 nicht als Zeichen einer erhöhten Nebenwirkung (systemische Zytokinfreisetzung) zu werten, sondern sich aus der spezifischen immunologischen Wirkung am Ort der Antigen-Bindung ergibt Ohne Kontakt zu HCT8 (Nebenwirkung) nimmt die Freisetzung von TNF-α demnach mit steigender Dexamethason-Konzentration weitaus stärker ab, als mit Kontakt zu HCT8 (spezifische Wirkung). D.h., es liegt eine sehr starke spezifische Wirkung (Freisetzung des Zytokins am Ort der Antigen-Bindung) vor.

In Betrachtung der Ergebnisse der Zytokinfreisetzung bei Verwendung des trifunktional bispezifischen Antikörpers anti-EpCAM x anti-CD3 zum einen bei gesunden Probanden (Figuren 11 bis 13), zum anderen bei Tumorpatienten (Figuren 14 bis 16) bleibt erfindungsgemäß festzustellen, dass die erzielten Ergebnisse bei den Tumorpatienten denen bei den gesunden Probanden entsprechen, wobei bei den Tumorpatienten die Auswirkungen des Glukokortikoids Dexamethason erfindungsgemäß stärker auf die unspezifische systemische Zytokinfreisetzung (Nebenwirkungen; ohne Kontakt zu HTCB) als auf die spezifische Wirkung (gegen EpCAM und CD-3) stattfindet, wodurch die Nebenwirkungen erfindungsgemäß stark herabgesetzt werden.

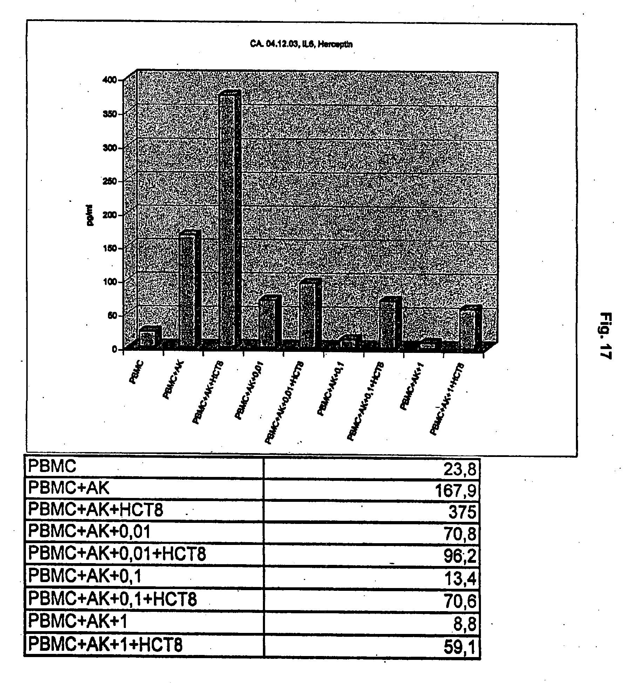

- Figuren 17 bis 18: zeigen graphische Darstellungen der Ergebnisse der Freisetzung von verschiedenen Zytokinen bei gesunden Probanden durch PBMC (bei einer Konzentration von 10⁶ Zellen/ml) nach Stimulation mit dem monoklonalen monospezifischen Antikörper Herceptin (AK) (bei einer Konzentration von 1 mg/ml) ohne bzw.in Gegenwart von HER2/neu positiven Tumorzellen (HCT8) (bei einer Konzentration von 5 x 10⁴ Zellen/ml). Auf den y-Achsen ist die Konzentration des jeweiligen Zytokins in pg/ml aufgetragen. Auf den x-Achsen sind die jeweiligen Stimulationsansätze der PBMC aufgetragen. Mit "0,01", "0,1" bzw. "1" wird die jeweilige Konzentration des zugegebenen Dexamethasons in µg/ml angegeben. Die Werte von unstimulierten PBMC als Kontrolle sind ebenfalls aufgetragen ("PBMC").
- Fig. 17: zeigt die Ergebnisse der Freisetzung von Interleukin 6 (IL-6). Bei Stimulation der PCMB mit dem Antikörper und Gabe von Dexamethason, nimmt die IL-6-Freisetzung ab, wobei sich die Abnahme mit steigender Konzentration von Dexamethason verstärkt. Die Freisetzung von IL-6 nimmt weitaus stärker ab, wenn kein gleichzeitiger Kontakt mit HCT8 vorliegt. Die absolut höhere Freisetzung des Zytokins bei Kontakt mit HCT8-Zellen ist kein Zeichen einer erhöhten Nebenwirkung (systemische Zytokinfreisetzung), sondern ergibt sich aus der spezifischen immunologischen Wirkung am Ort der Antigen-Bindung. Ohne Kontakt zu HCT8 (Nebenwirkung) nimmt die Freisetzung von IL-6 mit steigender Dexamethason-Konzentration stärker ab, als mit Kontakt zu HCT8 (spezifische Wirkung). Es liegt eine starke spezifische Wirkung (Freisetzung des Zytokins am Ort der Antigen-Bindung) vor.

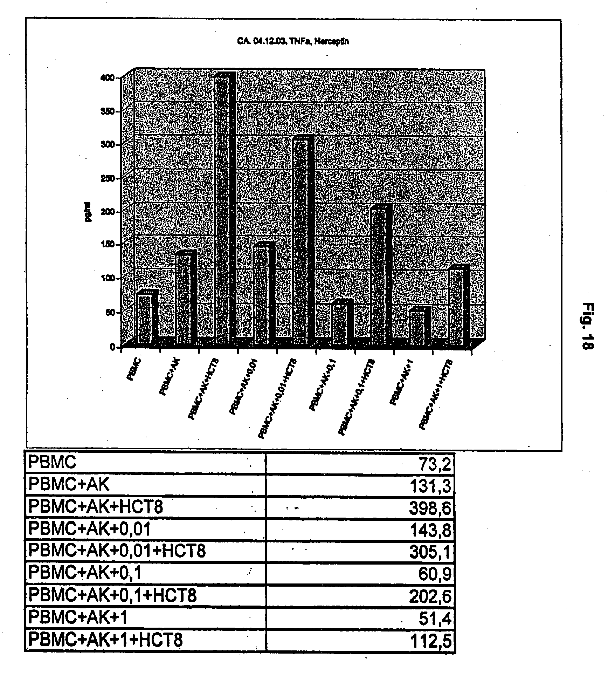

- Fig. 18: zeigt die Ergebnisse der Freisetzung von TNF-α. Bei Stimulation mit dem Antikörper und Gabe von Dexamethason nimmt die TNF-α-Freisetzung ab. Die Abnahme erfolgt umso stärker, je höher die Konzentration von Dexamethason ist. Die Freisetzung von TNF-α nimmt drastisch stärker ab, wenn kein gleichzeitiger Kontakt mit HCT8 vorliegt, wobei auch hier die absolut höhere Freisetzung des Zytokins bei Kontakt mit HCT8 kein Zeichen erhöhter Nebenwirkung (systemische Zytokinfreisetzung) ist, sondern sich aus der spezifischen immunologischen Wirkung am Ort der Antigen-Bindung ergibt. Ohne Kontakt zu HCT8 (Nebenwirkung) nimmt die Freisetzung von TNF-α demnach mit steigender Dexamethason-Konzentration signifikant stärker ab, als mit Kontakt zu HCT8 (spezifische Wirkung). D.h., es liegt eine sehr starke spezifische Wirkung (Freisetzung des Zytokins am Ort der Antigen-Bindung) vor.

Bei der Betrachtung der Ergebnisse der Freisetzung diverser Zytokine bei Stimulation von PBMC mit verschiedenen Antikörpern
(i) Versuchsansätze der Figuren 11 bis 16: Stimulation mit dem trifunktional bispezifischen Antikörper anti-EpCAM x anti-CD3;
(ii) Versuchsansätze der Figuren 17 bis 18: Stimulation mit dem monospezifisch monoklonalen Antikörper Herceptin
bleibt festzustellen, dass beide Antikörper, trotz ihrer verschiedenen Wirkmechanismen, im wesentlichen gleiche Effekte auf die Freisetzung verschiedener Zytokine, sowohl bei gesunden Probanden, als auch bei Tumorpatienten, zeigten.

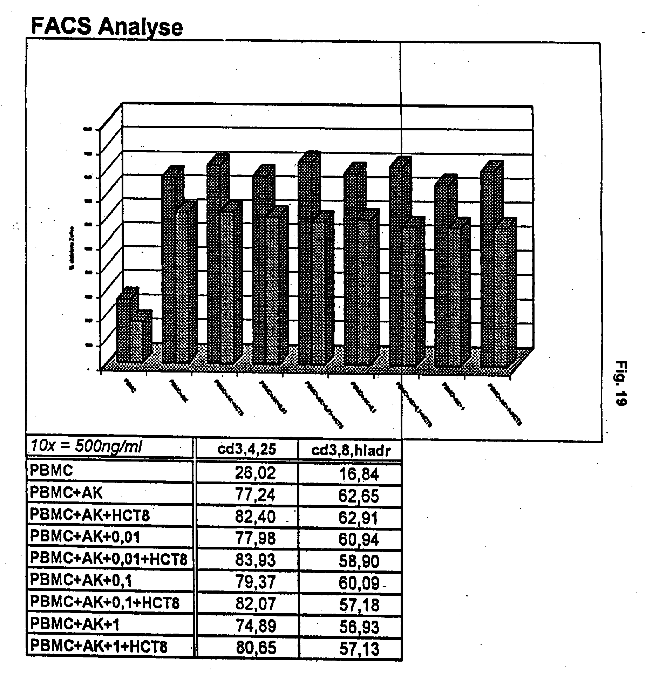

- Fig. 19: zeigt graphische Darstellungen der Ergebnisse von Messungen zur T-Zell-Aktivierung durch den trifunktionellen bispezifischen Antikörper mit der Spezifität anti-EpCAM x anti-CD3 bei einer Konzentration von 10⁶ Zellen/ml unter dem Einfluss der gleichzeitigen Gabe von Dexamethason (Konzentrationen von 0,01, 0,1 und 1 µg/ml). Es ist die Messung der Oberflächenmarker CD3/CD4/CD25 bzw. CD3/CD8/HLADR auf T-Helferzellen dargestellt. Hierbei stellen CD3/CD4 T-Helfer-Lymphozyten dar, wobei CD25 ist ein Aktivierungsmarker dieser Zellen ist CD3/CD8 stellen zytotoxische T-Lymphozyten dar, wobei HLADR ist ein Aktivierungsmarker dieser Zellen ist Auf y-Achse ist der prozentuale Anteil der CD3/CD4 (T-Helferzellen) bzw. CD3/CD8 (T-Killerzellen)-positiven T-Lymphozyten mit Expression der Aktivierungsmarker CD25 bzw. HLADR) aufgetragen. Auf der x-Achse sind die jeweiligen PBMC-Stimulationsansätze mit und ohne Kontakt zu HCTB-Zielzellen. (Konzentration 5 x 10⁴ Zellen/ml) sowie mit und ohne Gabe von Dexamethason in verschiedenen Konzentrationen aufgetragen, wobei die jeweils linke Säule die Messung CD3/CD4/CD25 betrifft und die jeweils rechte Säule die CD3/CD8/HLADR-Messung. Die Messungen erfolgten mittels FACS-Analyse. In der Tabelle unterhalb der Figur 19 sind die Messwerte (in Prozent) der jeweiligen Stimulationsansätze angegeben. Es ist zu erkennen, dass Dexamethason die T-Zell-Aktivierung nicht beeinflusst bei Stimulation mit dem trifunktionellen bispezifischen Antikörper unter Kontakt zu den HCT8-Zielzellen (diese Stimulation entspricht der spezifischen Stimulation mit Bindung des Ziel-Antigens. Dies belegen die unverändert hohen Messwerte in der Tabelle unterhalb der Figur 19.

Die folgenden Ausfuhrungsbeispiele erläutern die vorliegende Erfindung weiter, ohne sie einzuschränken.

### Ausfuhrungsbeispiel 1

### Gewinnung von PBMC

Es wurden mononukleäre Zellen aus dem peripheren Blut eines gesunden Freiwilligen mittels Zentrifugation über Ficoll (Dichte 1,068 g/cm³) gewonnen. Dazu wurde venöses heparinisiertes Blut über Ficoll geschichtet und bei 2000 UpM für 20 min zentrifugiert. Die über dem Ficoll befindliche Zellschicht wurde nach der Zentrifugation abpipettiert und mit PBS gewaschen.

### Stimulation von PBMC

Zur Stimulation wurden die PBMC bei einer Konzentration von 1 x 10⁶/ml verwendet. Die Stimulation der PBMC erfolgte mit einem intakten trifunktionellen bispezifischen Antikörper entweder der Spezifität anti-EpCAM x anti-CD3 bei einer Konzentration von 100 µg/ml oder der Spezifität anti-HER2/neu x anti-CD3 bei einer Konzentration von 1 mg/ml. Die Stimulation wurde für 24 h durch Inkubation bei 37°C durchgeführt. Die Stimulation erfolgte jeweils ohne Tumorzellen oder in Gegenwart von 5 x 10⁴ HCT-8-Tumorzellen (bezogen von ATCC; EpCAM-positiv) pro mL Zur Auswirkung des synthetischen Glukokortikoids Dexamethason (von Jenapharm®) wurden Stimulationsansätze ohne Glukokortikoid sowie Ansätze mit 0,01, 0,1, 1 bzw. 10 µg/ml durchgeführt.

### Bestimmung von Zytokinen

Nach der Stimulation erfolgte die Bestimmung der Konzentrationen der Zytokine IL-6, IL-10, TNF-α und IFN-γ im Überstand bei Platten mit 24 Vertiefungen jeweils mittels eines ELISA. Es wurden jeweils duplikate Messungen durchgeführt. Die ELISA erfolgten mittels stanardisierter Kits der Fa. R&D Systems entsprechend den Angaben des Herstellers.

### Ergebnisse

### Freisetzung von IL-6

IL-6 ist ein Zytokin, das durch immunologische Effektorzellen und Antigen-präsentierende Zellen direkt bei Eintreten einer immunologischen Reaktion ausgeschüttet wird. Die klinische Bedeutung von IL-6 als Zytokin im Serum ist dadurch bedingt, dass wenige Stunden nach Eintreten einer Entzündung bzw. einer Immunreaktion IL-6 ausgeschüttet wird und dessen Menge mit dem Ausmaß der Immunreaktion korreliert Im vorliegenden Ausführungsbeispiel zeigt sich bei Kontakt zu EpCAM-positiven Tumorzellen (HCT8) bei gleichzeitiger Zugabe von 0,01 bis 10 µg/ml Dexamethason eine Freisetzung von IL-6 mit Werten über 5000 pg/ml im Überstand (Fig. 1B). Diese Freisetzung entspricht der immunologischen Wirkung des EpCAM-spezifischen Antikörpers und stellt daher die gewünschte immunologische Wirkung dar. Diese starke IL-6-Freisetzung erfolgt nur von wenigen, direkt an der Reaktion beteiligten Immunzellen, die von der spezifischen Antigen-Antikörper-Wechselwirkung abhängig ist.

Im Gegensatz dazu zeigt sich bei einer Stimulation der PBMC mit Antikörpern ohne Kontakt zu HCT8-Tumorzellen eine IL-6-Freisetzung von über 9000 pg/ml (Fig. 1A). Diese Freisetzung tritt bei klinischer Applikation des immunstimulatorischen Antikörpers systemisch durch viele PBMC unabhängig von der gewünschten, spezifischen Wirkung auf. Durch die systemische IL-6-Freisetzung wird daher im Körper eine sehr große Menge an IL-6 erzeugt, welche schließlich für die starken Nebenwirkungen bis hin zu einem SIRS verantwortlich ist.

Durch Kombination des Antikörpers mit einem Glukokortikoid (vorliegend Dexamethason) wurde erfindungsgemäß festgestellt, dass die unspezifische IL-6-Freisetzung, die für die klinischen Nebenwirkungen immunstimulierender Antikörper wesentlich verantwortlich ist, durch Dexamethason vollständig reduziert wird. Gleichzeitig bleibt jedoch die IL-6-Freisetzung, welche nach Stimulation mit EpCAM-positiven Tumorzellen indiziert wird und somit der erwünschten spezifischen Wirkung des immunstimulatorischen Antikörpers entspricht, erhalten.

### Freisetzung von TNF-α

TNF-α ist ein TH1-Zytokin, das durch immunologische Effektorzellen (hier: PBMC) bei immunologischer Zerstörung von Zielzellen (hier: HCT8-Tumorzellen) ausgeschüttet wird. Es wurde im Vergleich zu einer unspezifischen Stimulation ohne Tumorzellen im vorliegenden Beispiel etwa dreifach erhöhte Werte von TNF-α nach Stimulation mit dem trifunktionellen bispezifschen Antikörper in Gegenwart von Zielzellen mit definiertem Antigen (EpCAM) gemessen (Fig. 2A und 2B). Diese deutliche. Erhöhung der TNF-α-Konzentration ergibt sich aus der Zielzell-abhängigen Freisetzung durch die PBMC bei der Tumorzell-Zerstörung. Wird der trifunktionelle bispezifsche Antikörper erfindungsgemäß mit einem Glukokortikoid, vorliegend Dexamethason, kombiniert, wird die auf unspezifischen Effekten beruhende TNF-α-Freisetzung vermindert bzw. bei entsprechend hoher Glukokortikoid-Konzentration vollständig reduziert. Somit lassen sich durch die erfindungsgemäße Verwendung von Glukokortikoiden die auf einer unspezifischen TNF-α-Freisetzung beruhenden klinischen Nebenwirkungen signifikant vermindern bzw. ausschalten. Im Gegensatz zur unspezifischen Zytokin-Freisetzung bleibt bei Zugabe von Dexamethason die Antigenabhängige und damit Zielzell-spezifische TNF-α-Freisetzung voll erhalten.

### Freisetzung von IFN-γ

IFN-γ ist ebenfalls ein TH1-Zytokin. Des weiteren wird IFN-γ von aktivierten spezifischen T-Lymphozyten nach Stimulation gegen ein Antigen ausgeschüttet, weshalb IFN-γ ein Marker für die T-Zell-vermittelte Antigen-spezifische Zielzell-Zerstörung ist. Daher wird IFN-γ nach einer Stimulation der PBMC mit dem trifunktionellen bispezifischen Antikörper in Abwesenheit von HCTB-Zielzellen nicht ausgeschüttet, da hierbei nur eine unspezifische Stimulation, die nicht gegen ein Antigen gerichtet ist, erfolgt (Fig. 3A). Bei der Stimulation in Gegenwart von HCT8-Zellen wurde eine signifikante IFN-γ-Freisetzung, die von der Stimulation gegen EpCAM durch den Antikörper hervorgerufen wird, gemessen (Fig. 3B). Diese Zielzell-spezifische IFN-γ-Freisetzung bleibt bei Glukokortikoid-Konzenttationen im vorliegenden Ausführungsbeispiel von unter 1 µg/ml im wesentlichen erhalten.

### Ausführungsbeispiel 2

Um den Einfluss von Glukokortikoid- auf die T-Zell-Aktivierung mittels Stimulation mit dem trifunktionellen bispezifischen Antikörper Anti-EpCAM x Anti-CD3 zu bestimmen, wurden Stimulationsexperimente, wie im vorstehenden Ausführungsbeispiel 1 angegeben, durchgeführt und danach die T-Zell-spezifische Aktivierungsmarker CD25, CD69 und HLA-DR bei CD3⁺/CD4⁺ bzw. CD3⁺/CD8⁺-T-Lymphozyten gemessen.

### FACS-Analyse

Die Durchführung der FACS-Analysen erfolgte unter Verwendung eines FACScalibur der Fa. Becton Dickinson. Es wurden entsprechend stimulierte PBMC mit FITC-, PE- und APCmarkierten Antikörpern der benötigten Spezifität markiert.

### Ergebnisse

Wie aus den Figuren 4A bis 4E hervorgeht, wurde bei einer Stimulation mit dem bispezifischen, trifunktionellen Antikörper keine Beeinflussung der T-Zell-Aktivierung durch das Glukokortikoid Dexamethason festgestellt.

### Ausführungsbeispiel 3

Mit Hilfe eines Zytotoxizitätstests wurde der Einfluss von Glukokortikoid auf die T-Zell-Toxizität untersucht. Die Gewinnung der PBMC und deren Stimulation wurde gemäß obigem Ausführungsbeispiel 1 durchgeführt.

### Zytotoxizitätstest

Die Messung der Zytotoxizität erfolgte mit Hilfe eines Fluoreszenz-Tests unter Verwendung des Farbstoffs BCECF-AM. Die Messung erfolgte mittels 2 h Release-Technik entsprechend der Vorgehensweise in Kolben et al. (1988) J. Immunol. Meth. 108: 255-264.

### Ergebnisse

Wie aus den Figuren 5A bis 5B hervorgeht, kann die Auswirkung von Dexamethason auf die durch den trifunktionellen, bispezifischen Antikörper hervorgerufene Zytotoxizität stimulierter PBMC wie folgt zusammengefaßt werden: Bei einer Konzentration von 0,01 mg/ml wurde kein Effekt von Dexamethason auf die Zytotoxizität der stimulierten PBMC beobachtet. Ab einer Konzentration von 0,1 µg/ml wurde eine signifikante Verminderung der Zytotoxizität unabhängig davon, ob HCTB-Tumorzellen während der Stimulation vorhanden waren oder nicht, beobachtet. Bei einer Stimulation in Gegenwart von HCT8 bleibt die prozentuale Zytotoxizität auf einem hohen Niveau konstant erhalten, wenn ein Verhältnis der Effektor-:Zielzellen von 40:1 und Konzentrationen von 0,1 bis 10 µg/ml Dexamethason verwendet wurden. Sind keine Zielzellen während der Stimulation der PBMC anwesend, fällt die prozentuale Zytotoxizität in Abhängigkeit von der Dexamethason-Konzentration weiter ab.

Die *in vitro-*Daten gemäß den Ausführungsbeispielen 1 bis 3 können daher wie folgt zusammengefaßt werden. Durch die Kombination von immunstimulierenden Antikörpern mit Glukokortikoiden kommt es zu einer Verminderung der unspezifischen Zytokinfreisetzung, ohne dass die erwünschte Antigen-spezifische Immunreaktion (Ausführungsbeispiel 1), T-Zell-Aktivierung (Ausführungsbeispiel 2) oder die Zytotoxizität der stimulierten Zellen (Ausführungsbeispiel 3) in gleichem Maße vermindert wird. Zwischen einer Konzentration von 0,1 und 1 µg/ml Dexamethason wird die unspezifische Reaktion stark vermindert oder vollständig ausgeschaltet. Im Gegensatz dazu wurde in diesem Glukokortikoid-Konzentrationsbereich *in vitro* die spezifische, gegen das Tumorzell-Antigen EpCAM gerichtete Immunaktivität nicht wesentlich beeinflusst.

### Ausführungsbeispiel 4

Ein Patient (46 Jahre, männlich) mit einem Magenkarzinom (Peritonealkarzinose; pT3 pN2 M1) wurde unter erfindungsgemäßer Verwendung eines Glukokortikoids mit dem trifunktionellen, bispezifischen Antikörper der Spezifität anti-EpCAM x anti-CD3 immuntherapiert. Die Therapie erfolgte nach Gastrektomie in 2000 und mehrfacher ineffektiver Chemotherapie des Tumors. Der Patient wies eine symptomatische Aszitesbildung auf. Die Prüfung der Tumorzellen im Aszites ergab eine starke EpCAM-Expression (EpCAM+++). Die Verabreichung des Antikörpers erfolgte intraperitoneal (i.p.) jeweils durch Infusion über 6 bis 10 h.

Zu Beginn der Therapie wurde dem Patienten der Antikörper in relativ geringen Dosen ohne Kombination mit Glukokortikoid verabreicht. Nach diesen 2 Monotherapie-Versuchen wurde die Antikörper-Dosis deutlich erhöht und gleichzeitig Dexamethason gegeben. In der nachstehenden Tabelle ist der Therapieverlauf unter Angabe der beobachteten Nebenwirkungen zusammengefaßt.

**Tab. 1: Therapieverlauf bei Patient mit Magenkarzinom**

| Tag | Therapie | Klinischer Verlauf |
|---|---|---|
| 0 | 10 E | Infusion über 6 h, 1000 ml Tuto ip |
| | | Prämedikation 2A Fenistil + 1000 mg benuron |
| | | Fieber bis 39.3 |
| | | Erbrechen |
| | | Bauchschmerzen, Gabe von Tramal |
| | | Abgeschlagenheit |
| 1 | | Fieberpexsistenz bis 39.0 |
| 2 | | |
| 3 | | Fieber 39.0 → Novalgin 1A 19.15 |
| 4 | 30 E | Infusion über 8 h, 1000 ml Tuto ip |
| | | Prämedikation 2A Fenistil + 1000 mg |
| | | Benuron |
| | | Therapieabbruch bei Fieber > 39.6 nach 6 h |
| | | Novalgin 1A |
| | | Schüttelfrost |
| | | Übelkeit |
| 5 | | Fieberpersistenz: |
| | | Novalgin 1A |
| | | Novalgin 30 ggt |
| | | Abgeschlagenheit |
| 6 | | |
| 7 | | |
| 8 | | Katheterwechsel |
| | | Erneute Aszitesmenge: 500 ml |
| 9 | | Geänderte Prämedikation |
| | | 2A Fenistil |
| | 100 E | 2A Tagamet |
| | | 40 mg Dexamethason |
| | | 1000 ml Tuto ip |
| | | Infusion über 8 h |
| | | Fieber bis maximal 39.0 |
| 10 | | |
| 11 | | |
| 12 | | Starke allergische Reaktion mit kompletter Hautrötung am Körperstamm |
| 13 | | |
| 14 | | |
| 15 | 100 E | 2A Fenistil |
| | | 2A Tagamet |
| | | 10 mg Dexamethason |
| | | 1000 ml Tuto ip |
| | | Infusion über 8 h |
| | | Kein Fieber |
| | | Keine NW |
| 16 | | |
| 17 | | |
| 18 | | |
| 19 | 200 E | 2A Fenistil |
| | | 2A Tagamet |
| | | 10 mg Dexamethason |
| | | 1000 ml Tuto ip |
| | | Infusion über 10 h |
| | | Kein Fieber |
| | | Keine NW |
| 20 | | |
| 21 | | |
| 22 | | |
| 23 | 500 E | 2A Fenistil |
| | | 2A Tagamet |
| | | 10 mg Dexamethason |
| | | 1000 ml Tuto ip |
| | | Infusion über 8 h |
| | | Kein Fieber |
| | | Keine NW |

| | | |
|---|---|---|
| E: µg i.p. trifunkioneller bispezifischer Antikörper anti-EpCAM x anti-CD3 NW: Nebenwirkungen | | |

Wie aus der Tab. 1 hervorgeht, litt der Patient bei Monotherapie mit dem immunstimulatorischen Antikörper bei einer Dosis von 10 mg bis 30 mg unter starken Nebenwirkungen, insbesondere Erbrechen, Bauchschmerzen, Unwohlsein sowie hohem Fieber. Wurde jedoch der Antikörper mit Dexamethason kombiniert, konnte die Antikörper-Dosierung auf 500 mg i.p. gesteigert werden, ohne dass Fieber oder andere Nebenwirkungen auftraten. Die immunologischen Labor-Parameter bestätigten die starke, spezifisch gegen die Tumorzellen gerichtete immunstimulatorische Wirkung des Antikörpers (Fig. 6). Leber- und Pankreas-Werte waren unter der erfindungsgemäßen Kombinationstherapie weitgehend unauffällig (Fig. 7). Nach Abschluß der erfindungsgemäßen Therapie wurde ein vollständiges Verschwinden von malignem Aszites sowie im Therapieverlauf eine vollständige Eliminierung der malignen Tumorzellen im Aszites festgestellt.

### Ausführungsbeispiel 5

In einem weiteren erfindungsgemäßen Beispiel wurde eine Patientin (68 Jahre) mit einem Adenokarzinom (Sigma pT3 pN2 M1) und diffuser Lebermetastasierung mit dem trifunktionellen bispezifischen Antikörper anti-EpCAM x anti-CD3 in Kombination mit dem Glukokortikoid Dexamethason immuntherapiert Zuvor wurde festgestellt, dass die Zellen der Lebermetastasen zu 80% EpCAM-positiv waren. Bei der vorliegenden Therapie erfolgte die Applikation des Antikörpers über einen selektiven Katheter über die A. hepatica dextra als Beispiel einer selektiven Verabreichung in ein Organ (vorliegend intrahepatisch) bzw. systemisch (nach Durchströmen der Leber). Die Verabreichung von Dexamethason erfolgte jeweils als Prämedikation vor der Verabreichung des immunstimulatorischen Antikörpers.

Die Therapie und deren Verlauf sind in der nachfolgenden Tab. 2 angegeben.

**Tab. 2: Therapieverlauf bei Patientin mit Adeno-Karzinom und diffuser Lebermetastasierung**

| Tag | Therapie | Bemerkung |
|---|---|---|
| 0 | 1 µg | Applikation über selektiven |
| | | arteriellen Port in A. hepatica dextra über 8 h |
| | | Prämedikation: Dexa 40 mg, 2A Fenistil, 2A Ranitidin |
| | | Kein Fieber, keine Nebenwirkungen |
| 1 | | |
| 2 | | |
| 3 | 5 µg | Applikation über selektiven arteriellen Port in A. hepatica dextra über 8h |
| | | Prämedikation: Dexa 20 mg, 2A Fenistil, 2A Ranitidin |
| | | Fieber bis 39,8°C 9 h nach Therapiestart |
| | | Medikation mit Metamizol 1A i.v. |
| | | Temperaturnormalisietung innerhalb von 4 h |
| 4 | | |
| 5 | | |
| 6 | 20 µg | Applikation über selektiven arteriellen Port in A. hepatica dextra über 8 h |
| | | Prämedikation: Dexa 20 mg, 2A Fenistil, 2A Ranitidin |
| | | Fieber bis 39,6°C 10 h nach Therapiestart |
| | | Medikation mit Metamizol 1A i.v. Temperaturnormalisierung innerhalb von 4 h |
| 7 | | |
| 8 | | |
| 9 | | |
| 10 | 4 µg | Applikation über selektiven arteriellen Port in A. hepatica dextra über 10 h |
| | | Prämedikation: Dexa 20 mg, 2A Fenistil, 2A Ranitidin |
| | | Fieber bis 40,1°C 6 h nach Therapiestart |
| | | Medikation mit Metamizol 2A i.v. |
| | | Temperatumormalisierung innerhalb von 10 h |
| | | Schüttelfrost/Gliederschmerzen |

Unter der erfindungsgemäßen Kombination des Glukokortikoids mit dem immuntherapeutischen Antikörper kam es nur zu einem vorübergehenden Fieber, wobei eine Temperatumormalisierung spätestens innerhalb von 10 h nach dem Erreichen der Maximaltemperatur erfolgte. Trotz der sehr starken Dosis-Erhöhung des Antikörpers um den Faktor 40 (von 1 µg auf 40 µg) während der Therapie wurde somit keine dieser immensen Steigerung entsprechende Verstärkung der Nebenwirkungen des immunstimulatorischen Antikörpers festgestellt, was auf die erfindungsgemäße Verabreichung des Glukokortikoids zurückzuführen ist. Die Verabreichung von Dexamethason hatte keinen bedeutsamen negativen Einfluss auf die immunstimulierende Wirkung des Antikörpers. Dies wird durch einen systemischen Anstieg des löslichen IL-2-Rezeptors und der TNF-Rezeptoren p55 und p75 dokumentiert (Fig. 8). Demgegenüber konnten trotz der Dosissteigerung bezüglich des Antikörpers auf das 40fache keine signifikanten Nebenwirkungen festgestellt werden. Dies bestätigen insbesondere auch die Pankreas- und Leber-spezifischen Labor-Parameter (Fig. 9).

## Patentansprüche

1. Ein oder mehrere Glukokortikoid(e) zur Verminderung der unspezifischen Zytokinfreisetzung bei der Behandlung von Krebserkrankungen und Tumorerkrankungen mit einem oder mehreren immunstimulierenden trifunktionellen, bispezifischen Antikörper(n) (trAK).

2. Glukokortikoid(e) zur Verwendung nach Anspruch 1, wobei das Glukokortikoid ein synthetisches Glukokortikoid ist.

3. Glukokortikoid(e) zur Verwendung nach Anspruch 2, wobei das synthetische Glukokortikoid aus der Gruppe, bestehend aus Prednison, Prednisolon, Methylprednisolon, Triamcinolon, Betamethason, Dexamethason, Cortisonacetat, Prednyliden, Deflazacort, Cloprednol, Fluocortolon und Budenosid, ausgewählt ist.

4. Glukokortikoid(e) zur Verwendung nach einem der Ansprüche 1 bis 3, wobei die Krebserkrankung aus der Gruppe, bestehend aus Magenkarzinom, Adenokarzinom, malignes Melanom, Kolonkarzinom, Pankreaskarzinom, Ovarialkarzinom, Uteruskarzinom, Hepatozelluläres Karzinom, allen histologischen Typen des Bronchialkarzinoms, Lymphomen, Sarkomen, Blastomen und gastrointestinaler Stromatumor (GIST) ausgewählt ist.

5. Glukokortikoid(e) zur Verwendung nach einem der Ansprüche 1 bis 4, wobei der Antikörper ein monoklonaler Antikörper, chimärer Antiköper, und/oder humanisierter Antikörper ist.

6. Glukokortikoid(e) zur Verwendung nach einem der Ansprüche 1 bis 6, wobei der Antikörper jeweils mindestens eine Spezifität gegen Tumorantigen und gegen einen CD-Marker aufweist.

7. Glukokortikoid(e) zur Verwendung nach Anspruch 6, wobei der CD-Marker aus der Gruppe, bestehend aus CD2, CD3, CD4, CD5, CD6, CD8, CD28 und CD44, ausgewählt ist.

8. Glukokortikoid(e) zur Verwendung nach Anspruch 6 oder 7, wobei das Tumorantigen aus der Gruppe, bestehend aus EpCAM, HER2/neu, HER3/neu, G250, CEA, MAGE, VEGF, GD3, EGFR, αVβ3-Integrin, HLA, HLA-DR, ASC, CD1, CD2, CD4, CD6, CD7, CD8, CD11, CD13, CD14, CD19, CD20, CD21, CD22, CD23, CD24, CD33, CD40, CD41, CD52, c-erb-2, CALLA, MHCII, CD44v3, CD44v6, CD117, p97, Gangliosid GD2, GD3, C215, Antigen des Antikörpers 9.2.27, Antigen des Antikörpers NE150 und Antigen des Antikörpers CA125, ausgewählt ist.

9. Glukokortikoid(e) zur Verwendung nach einem der Ansprüche 6 bis 8, wobei der Antikörper die Spezifität anti-EpCAM x anti-CD3 oder anti-HER2/neu x anti-CD3 besitzt.

10. Glukokortikoid(e) zur Verwendung nach einem der Ansprüche 1 bis 9, wobei der Antikörper und/oder das Glukokortikoid intraperitoneal, intravenös, intraarteriell, intramuskulär, intradermal, subkutan, intratumoral oder selektiv in ein definiertes Organ verabreicht wird.

11. Glukokortikoid(e) zur Verwendung nach Anspruch 10, wobei die selektive Verabreichung in ein definiertes Organ über einen Katheter in das versorgende Gefäß erfolgt.

12. Glukokortikoid(e) zur Verwendung nach Anspruch 11, wobei die Verabreichung in die Leber über einen Katheter in die A. hepatica erfolgt.

13. Glukokortikoid(e) zur Verwendung nach einem der Ansprüche 1 bis 12, wobei das Glukokortikoid und der Antikörper gleichzeitig verabreicht werden.

14. Pharmazeutische Zusammensetzung umfassend mindestens einen immunstimulierenden trifunktionellen, bispezifischen Antikörper und mindestens ein Glukokortikoid, wobei der Antikörper die Spezifität anti-EpCAM x anti-CD3 oder anti-HER2/neu x anti-CD3 besitzt.

15. Pharmazeutische Zusammensetzung zur Verminderung der unspezifischen Zytokinfreisetzung bei der Behandlung von Krebserkrankungen und Tumorerkrankungen mit einem oder mehreren immunstimulierenden trifunktionellen, bispezifischen Antikörper(n) (trAK),
enthaltend mindestens einen immunstimulierenden trifunktionellen, bispezifischen Antikörper und mindestens ein Glukokortikoid, gegebenenfalls in Verbindung mit einem oder mehreren pharmazeutisch verträglichen Trägern und/oder Hilfsstoffen.

16. Pharmazeutische Zusammensetzung nach Anspruch 14 oder zur Verwendung nach Anspruch 15, wobei das Glukokortikoid ein synthetisches Glukokortikoid ist.

17. Pharmazeutische Zusammensetzung oder pharmazeutische Zusammensetzung zur Verwendung nach Anspruch 16, wobei das synthetische Glukokortikoid aus der Gruppe, bestehend aus Prednison, Prednisolon, Methylprednisolon, Triamcinolon, Betamethason, Dexamethason, Cortisonacetat, Prednyliden, Deflazacort, Cloprednol, Fluocortolon und Budenosid, ausgewählt ist.

18. Pharmazeutische Zusammensetzung oder pharmazeutische Zusammensetzung zur Verwendung nach einem der Ansprüche 14 bis 17, wobei der Antikörper ein monoklonaler Antikörper, chimärer Antiköper und/oder humanisierter Antikörper ist.

19. Pharmazeutische Zusammensetzung oder pharmazeutische Zusammensetzung zur Verwendung nach einem der Ansprüche 15 bis 17, wobei der Antikörper jeweils mindestens eine Spezifität gegen Tumorantigen und gegen einen CD-Marker aufweist.

20. Pharmazeutische Zusammensetzung oder pharmazeutische Zusammensetzung zur Verwendung nach Anspruch 19, wobei der CD-Marker aus der Gruppe, bestehend aus CD2, CD3, CD4, CD5, CD6, CD8, CD28 und CD44, ausgewählt ist.

21. Pharmazeutische Zusammensetzung oder pharmazeutische Zusammensetzung zur Verwendung nach Anspruch 19 oder 20, wobei das Tumorantigen aus der Gruppe, bestehend aus EpCAM, HER2/neu, HER3/neu, G250, CEA, MAGE, VEGF, GD3, EGFR, αVβ3-Integrin, HLA, HLA-DR, ASC, CD1, CD2, CD4, CD6, CD7, CD8, CD11, CD13, CD14, CD19, CD20, CD21, CD22, CD23, CD24, CD33, CD40, CD41, CD52, c-erb-2, CALLA, MHCII, CD44v3, CD44v6, p97, Gangliosid GD2, GD3, C215, Antigen des Antikörpers 9.2.27, Antigen des Antikörpers NE150 und Antigen des Antikörpers CA125, ausgewählt ist.

22. Pharmazeutische Zusammensetzung oder pharmazeutische Zusammensetzung zur Verwendung nach einem der Ansprüche 19 bis 21, wobei der Antikörper die Spezifität anti-EpCAM x anti-CD3 oder anti-HER2/neu x anti-CD3 besitzt.

23. Pharmazeutische Zusammensetzung nach Anspruch 14 zur Behandlung von Erkrankungen, ausgewählt aus der Gruppe, bestehend aus Krebserkrankungen und Tumorerkrankungen.

24. Pharmazeutische Zusammensetzung zur Verwendung nach einem der Ansprüche 15 bis 23, wobei die Krebserkrankung aus der Gruppe, bestehend aus Magenkarzinom, Adenokarzinom, malignes Melanom, Kolonkarzinom, Pankreaskarzinom, Ovarialkarzinom, Uteruskarzinom, Hepatozelluläres Karzinom, allen histologischen Typen des Bronchialkarzinoms, Lymphomen, Sarkomen und Blastomen, ausgewählt ist.

25. Kit enthaltend mindestens einen immunstimulierenden trifunktionellen, bispezifischen Antikörper (trAK) und mindestens ein Glukokortikoid, wobei der Antikörper die Spezifität anti-EpCAM x anti-CD3 oder anti-HER2/neu x anti-CD3 besitzt, wobei der mindestens eine immunstimulierende trifunktionelle bispezifische Antikörper und das mindestens eine Glukokortikoid voneinander getrennt sind.

26. Kit nach Anspruch 25, wobei das Glukokortikoid aus der Gruppe, bestehend aus Prednison, Prednisolon, Methylprednisolon, Triamcinolon, Betamethason, Dexamethason, Cortisonacetat, Prednyliden, Deflazacort, Cloprednol, Fluocortolon und Budenosid, ausgewählt ist.

## Claims

1. One or more glucocorticoid(s) for reducing the non-specific release of cytokine on the treatment of cancer diseases and tumor diseases with one or more immunostimulating trifunctional, bispecific antibody/antibodies (trAB).

2. Glucocorticoid(s) for use according to Claim 1, wherein the glucocorticoid is a synthetic glucocorticoid.

3. Glucocorticoid(s) for use according to Claim 2, wherein the synthetic glucocorticoid is selected from the group consisting of prednisone, prednisolone, methylprednisolone, triamcinolone, betamethasone, dexamethasone, cortisone acetate, prednylidene, deflazacort, cloprednol, fluocortolone and budenoside.

4. Glucocorticoid(s) for use according to any of Claims 1 to 3, wherein the cancer disease is selected from the group consisting of gastric carcinoma, adenocarcinoma, malignant melanoma, colonic carcinoma, pancreatic carcinoma, ovarian carcinoma, uterine carcinoma, hepatocellular carcinoma, all histological types of bronchial carcinoma, lymphomas, sarcomas, blastomas and gastrointestinal stromal tumor (GIST).

5. Glucocorticoid(s) for use according to any of Claims 1 to 4, wherein the antibody is a monoclonal antibody, a chimeric antibody and/or a humanized antibody.

6. Glucocorticoid(s) for use according to any of Claims 1 to 5, wherein the antibody comprises each at least a specificity against a tumor antigen and a specificity against a CD marker.

7. Glucocorticoid(s) for use according to Claim 6, wherein The CD marker is selected from the group consisting of CD2, CD3, CD4, CD5, CD6, CD8, CD28 and CD44.

8. Glucocorticoid(s) for use according to Claim 6 or 7, wherein the tumor antigen is selected from the group consisting of EpCAM, HER2/neu, HER3/neu, G250, CEA, MAGE, VEGF, EGFR, αVβ3-integrin, HLA, HLA-DR, ASC, CD1, CD2, CD4, CD6, CD7, CD8, CD11, CD13, CD14, CD19, CD20, CD21, CD22, CD23, CD24, CD33, CD40, CD41, CD52, c-erb-2, CALLA, MHCII, CD44v3, CD44v6, CD117, p97, ganglioside GD2, GD3, C215, antigen of the antibody 9.2.27, antigen of the antibody NE150 and antigen of the antibody CA125.

9. Glucocorticoid(s) for use according to any of Claims 6 to 8, wherein the antibody has the specificity anti-EpCAM x anti-CD3 or anti-HER2/neu x anti-CD3.

10. Glucocorticoid(s) for use according to any of Claims 1 to 9, wherein the antibody and/or the glucocorticoid is to be administered intraperitoneally, intravenously, intraarterially, intramuscularly, intradermally, subcutaneously, intratumorally or selectively into a defined organ.

11. Glucocorticoid(s) for use according to Claim 10, wherein the selective administration into a defined organ is via a catheter into the supplying blood vessel.

12. Glucocorticoid(s) for use according to Claim 11, wherein the administration into the liver is via a catheter into the A. hepatica.

13. Glucocorticoid(s) for use according to any of Claims 1 to 12, wherein the glucocorticoid and the antibody are administered simultaneously.

14. Pharmaceutical composition comprising at least one immunostimulating trifunctional, bispecific antibody (trAB) and at least one glucocorticoid, wherein the antibody has the specificity anti-EpCAM x anti-CD3 or anti-HER2/neu x anti-CD3.

15. Pharmaceutical composition for reducing the non-specific release of cytokine in the treatment of cancer diseases and tumor diseases with one or more immunostimulating trifunctional, bispecific antibody/antibodies (trAB), comprising at least one immunostimulating trifunctional, bispecific antibody and at least one glucocorticoid, if necessary in combination with one or more pharmaceutically compatible carriers and/or adjuvants.

16. Pharmaceutical composition according to Claim 14 or for use according to claim 15, wherein the glucocorticoid is a synthetic glucocorticoid.

17. Pharmaceutical composition or pharmaceutical composition for use according to Claim 16, wherein the synthetic glucocorticoid is selected from the group consisting of prednisone, prednisolone, methylprednisolone, triamcinolone, betamethasone, dexamethasone, cortisone acetate, prednylidene, deflazacort, cloprednol, fluocortolone and budenoside.

18. Pharmaceutical composition or pharmaceutical composition for use according to any of Claims 14 to 17, wherein the antibody is a monoclonal antibody, a chimeric antibody and/or a humanized antibody.

19. Pharmaceutical composition or pharmaceutical composition for use according to any of Claims 15 to 17, wherein the antibody comprises each at least a specificity against a tumor antigen and a specificity against a CD marker.

20. Pharmaceutical composition or pharmaceutical composition for use according to Claim 19, wherein the CD marker is selected from the group consisting of CD2, CD3, CD4, CD5, CD6, CD8, CD28 and CD44.

21. Pharmaceutical composition or pharmaceutical composition for use according to Claim 19 or 20, wherein the tumor antigen is selected from the group consisting of EpCAM, HER2/neu, HER3/neu, G250, CEA, MAGE, VEGF, GD3, EGFR, αVβ3-integrin, HLA, HLA-DR, ASC, CD1, CD2, CD4, CD6, CD7, CD8, CD11, CD13, CD14, CD19, CD20, CD21, CD22, CD23, CD24, CD33, CD40, CD41, CD52, c-erb-2, CALLA, MHCII, CD44v3, CD44v6, p97, ganglioside GD2, GD3, C215, antigen of the antibody 9.2.27, antigen of the antibody NE150 and antigen of the antibody CA125.

22. Pharmaceutical composition or pharmaceutical composition for use according to any of Claims 19 to 21, wherein the antibody has the specificity anti-EpCAM x anti-CD3 or anti-HER2/neu x anti-CD3.

23. Pharmaceutical composition according to claim 14 for the treatment of diseases selected from the group consisting of cancer diseases and tumor diseases.

24. Pharmaceutical composition for use according to any of Claims 15 to 23 wherein the cancer disease is selected from the group consisting of gastric carcinoma, adenocarcinoma, malignant melanoma, colonic carcinoma, pancreatic carcinoma, ovarian carcinoma, uterine carcinoma, hepatocellular carcinoma, all histological types of bronchial carcinoma, lymphomas, sarcomas and blastomas.

25. Kit comprising at least one immunostimulating trifunctional, bispecific antibody (trAB) and at least one glucocorticoid wherein the antibody has the specificity anti-EpCAM x anti-CD3 or anti-HER2/neu x anti-CD3, wherein the at least one immunostimulating trifunctional, bispecific antibody and the at least one glucocorticoid are separated from each other.

26. Kit according to Claim 25, wherein the glucocorticoid is selected from the group consisting of prednisone, prednisolone, methylprednisolone, triamcinolone, betamethasone, dexamethasone, cortisone acetate, prednylidene, deflazacort, cloprednol, fluocortolone and budenoside.

## Revendications

1. Un ou plusieurs glucocorticoïdes pour réduire la libération de cytokines non spécifique dans le traitement des maladies cancéreuses et des maladies tumorales avec un ou plusieurs anticorps bispécifiques trifonctionnels (trAC) immunostimulants.

2. Glucocorticoïde(s) destiné(s) à être utilisé(s) selon la revendication 1 où le glucocorticoïde est un glucocorticoïde synthétique.

3. Glucocorticoïde(s) destiné(s) à être utilisé(s) selon la revendication 2 où le glucocorticoïde synthétique est choisi dans le groupe consistant en prednisone, prednisolone, méthylprednisolone, triamcinolone, bêtaméthasone, dexaméthasone, acétate de cortisone, prednylidène, deflazacort, cloprednol, fluocortolone et budésonide.

4. Glucocorticoïde(s) destiné(s) à être utilisé(s) selon l'une des revendications 1 à 3 où la maladie cancéreuse est choisie dans le groupe consistant en cancer de l'estomac, adénocarcinome, mélanome malin, cancer du côlon, cancer du pancréas, cancer ovarien, cancer de l'utérus, carcinome hépatocellulaire, tous les types histologiques du cancer bronchique, lymphomes, sarcomes, blastomes et tumeur stromale gastro-intestinale (GIST).

5. Glucocorticoïde(s) destiné(s) à être utilisé(s) selon l'une des revendications 1 à 4 où l'anticorps est un anticorps monoclonal, un anticorps chimérique et/ou un anticorps humanisé.

6. Glucocorticoïde(s) destiné(s) à être utilisé(s) selon l'une des revendications 1 à 5 où l'anticorps présente au moins une spécificité contre un antigène tumoral et au moins une spécificité contre un marqueur CD.

7. Glucocorticoïde(s) destiné(s) à être utilisé(s) selon la revendication 6 où le marqueur CD est choisi dans le groupe consistant en CD2, CD3, CD4, CD5, CD6, CD8, CD28 et CD44.

8. Glucocorticoïde(s) destiné(s) à être utilisé(s) selon la revendication 6 ou 7 où l'antigène tumoral est choisi dans le groupe consistant en EpCAM, HER2/neu, HER3/neu, G250, CEA, MAGE, VEGF, EGFR, intégrine αVβ3, HLA, HLA-DR, ASC, CD1, CD2, CD4, CD6, CD7, CD8, CD11, CD13, CD14, CD19, CD20, CD21, CD22, CD23, CD24, CD33, CD40, CD41, CD52, c-erb-2, CALLA, MHCII, CD44v3, CD44v6, CD 117, p97, ganglioside GD2, GD3, C215, antigène de l'anticorps 9.2.27, antigène de l'anticorps NE150 et antigène de l'anticorps CA125.

9. Glucocorticoïde(s) destiné(s) à être utilisé(s) selon l'une des revendications 6 à 8 où l'anticorps possède la spécificité anti-EpCAM x anti-CD3 ou anti-HER2/neu x anti-CD3.

10. Glucocorticoïde(s) destiné(s) à être utilisé(s) selon l'une des revendications 1 à 9 où l'anticorps et/ou le glucocorticoïde est administré par voie intrapéritonéale, intraveineuse, intra-artérielle, intramusculaire, intradermique, sous-cutanée, intratumorale ou sélectivement dans un organe défini.

11. Glucocorticoïde(s) destiné(s) à être utilisé(s) selon la revendication 10 où l'administration sélective a lieu dans un organe défini par le biais d'un cathéter dans le vaisseau nourricier.

12. Glucocorticoïde(s) destiné(s) à être utilisé(s) selon la revendication 11 où l'administration a lieu dans le foie par le biais d'un cathéter dans l'artère hépatique.

13. Glucocorticoïde(s) destiné(s) à être utilisé(s) selon l'une des revendications 1 à 12 où le glucocorticoïde et l'anticorps sont administrés en même temps.

14. Composition pharmaceutique comprenant au moins un anticorps bispécifique trifonctionnel immunostimulant et au moins un glucocorticoïde, où l'anticorps possède la spécificité anti-EpCAM x anti-CD3 ou anti-HER2/neu x anti-CD3.

15. Composition pharmaceutique pour réduire la libération de cytokines non spécifique dans le traitement des maladies cancéreuses et des maladies tumorales avec un ou plusieurs anticorps bispécifiques trifonctionnels (trAC) immunostimulants, contenant au moins un anticorps bispécifique trifonctionnel immunostimulant et au moins un glucocorticoïde, éventuellement en liaison avec un ou plusieurs vecteurs et/ou adjuvants pharmaceutiquement acceptables.

16. Composition pharmaceutique selon la revendication 14 ou destinée à être utilisée selon la revendication 15 où le glucocorticoïde est un glucocorticoïde synthétique.

17. Composition pharmaceutique ou composition pharmaceutique destinée à être utilisée selon la revendication 16 où le glucocorticoïde synthétique est choisi dans le groupe consistant en prednisone, prednisolone, méthylprednisolone, triamcinolone, bêtaméthasone, dexaméthasone, acétate de cortisone, prednylidène, deflazacort, cloprednol, fluocortolone et budésonide.

18. Composition pharmaceutique ou composition pharmaceutique destinée à être utilisée selon l'une des revendications 14 à 17 où l'anticorps est un anticorps monoclonal, un anticorps chimérique et/ou un anticorps humanisé.

19. Composition pharmaceutique ou composition pharmaceutique destinée à être utilisée selon l'une des revendications 15 à 17 où l'anticorps présente au moins une spécificité contre un antigène tumoral et au moins une spécificité contre un marqueur CD.

20. Composition pharmaceutique ou composition pharmaceutique destinée à être utilisée selon la revendication 19 où le marqueur CD est choisi dans le groupe consistant en CD2, CD3, CD4, CD5, CD6, CD8, CD28 et CD44.

21. Composition pharmaceutique ou composition pharmaceutique destinée à être utilisée selon la revendication 19 ou 20 où l'antigène tumoral est choisi dans le groupe consistant en EpCAM, HER2/neu, HER3/neu, G250, CEA, MAGE, VEGF, GD3, EGFR, intégrine αVβ3, HLA, HLA-DR, ASC, CD1, CD2, CD4, CD6, CD7, CD8, CD11, CD13, CD14, CD 19, CD20, CD21, CD22, CD23, CD24, CD33, CD40, CD41, CD52, c-erb-2, CALLA, MHCII, CD44v3, CD44v6, p97, ganglioside GD2, GD3, C215, antigène de l'anticorps 9.2.27, antigène de l'anticorps NE150 et antigène de l'anticorps CA125.

22. Composition pharmaceutique ou composition pharmaceutique destinée à être utilisée selon l'une des revendications 19 à 21 où l'anticorps possède la spécificité anti-EpCAM x anti-CD3 ou anti-HER2/neu x anti-CD3.

23. Composition pharmaceutique selon la revendication 14 pour le traitement des maladies choisies dans le groupe consistant en les maladies cancéreuses et les maladies tumorales.

24. Composition pharmaceutique destinée à être utilisée selon l'une des revendications 15 à 23 où la maladie cancéreuse est choisie dans le groupe consistant en cancer de l'estomac, adénocarcinome, mélanome malin, cancer du côlon, cancer du pancréas, cancer ovarien, cancer de l'utérus, carcinome hépatocellulaire, tous les types histologiques du cancer bronchique, lymphomes, sarcomes et blastomes.

25. Kit contenant au moins un anticorps bispécifique trifonctionnel (trAC) immunostimulant et au moins un glucocorticoïde, où l'anticorps possède la spécificité anti-EpCAM x anti-CD3 ou anti-HER2/neu x anti-CD3, où le au moins un anticorps bispécifique trifonctionnel immunostimulant et le au moins un glucocorticoïde sont séparés l'un de l'autre.

26. Kit selon la revendication 25 où le glucocorticoïde est choisi dans le groupe consistant en prednisone, prednisolone, méthylprednisolone, triamcinolone, bêtaméthasone, dexaméthasone, acétate de cortisone, prednylidène, deflazacort, cloprednol, fluocortolone et budésonide.
